# EUROPEAN PATENT APPLICATION

(11) **EP 3 978 630 A1**
(43) Date of publication of application: **06.04.2022**
(21) Application number: 20306152.8
(22) Date of filing: 05.10.2020
(51) Int. Cl.: C12Q 1/6886

(54) **METHOD FOR PREDICTING THE EFFICIENCY OF A TREATMENT STIMULATING AN IFN-BETA DEPENDENT ADAPTIVE IMMUNE RESPONSE VIA DETECTION OF A SINGLE NUCLEOTIDE POLYMORPHISM**

(71) Applicant: Commissariat à l'Energie Atomique et aux Energies Alternatives, 75015 Paris (FR)
(72) Inventor: ROUSSELET, Germain, 92260 Fontenay aux Roses (FR); ASSOUVIE, Anaïs, 92260 Fontenay aux Roses (FR)
(74) Representative: Regimbeau

(57) **Abstract**

The present invention relates to a method for predicting the efficiency of a treatment stimulating an IFN-ß dependent adaptive immune response, comprising a step of detecting the rs12553564 single nucleotide polymorphism (SNP), or an SNP in high linkage disequilibrium with same, said SNP being selected from rs12551341, rs2275888, and rs10811449, in a biological sample of a subject in need thereof.

## Description

### Field of the invention

The present invention relates to the field of Interferon β (IFN-B) adaptive immune response establishment and, more precisely, to a method of predicting if an Interferon β (IFN-β) adaptive immune response is likely to occur after a conventional therapy.

### Background of the invention

IFN-B is a cytokine that induces a global antiviral proteome, and regulates the adaptive immune response to infections and tumors. In particular, it is an essential step of the antiviral response (1). The transcription of the IFN-B coding gene, *IFNB1,* is rapidly induced upon viral infection through multiple pathways sensing virus-derived nucleic acids (2). The released IFN-B protein is able to induce the expression of antiviral proteins encoded by Interferon Stimulated Genes (ISGs) (3, 4), that interfere with the infection of the cell by other viruses, hence the name interferon. In addition, IFN-B targets immune cells, facilitating the induction of an efficient adaptive immune response (2). It promotes the activation and the T cell stimulatory capacity of dendritic cells (5, 6), and has direct co-stimulatory properties on T cells, in particular by stimulating their proliferation once they have been activated by engagement of the T cell receptor and of co-stimulatory receptors (7).

The pioneering work performed in the laboratories of T. Maniatis and D. Thanos notably allowed for the identification of the "enhanceosome," a promoter proximal structure that serves as a docking site for the cooperative binding of several transcription factors involved in *IFNB1* transcriptional control, including NF-κB (p50:relA), ATF2:c-jun, and IRF3/IRF7 (8-10). Several regions upstream or downstream from the *IFNB1* gene have been described to positively or negatively regulate *IFNB1* transcription by fixing factors such as NF-κB (11), XBP-1 (12), YY1/2 (13, 14), or β-catenin (15). In particular, Banerjee et al. identified a region located 20 kb upstream from the human *IFNB1* gene that loops to the *IFNB1* promoter, binds phospho-IRF3, and is required for *IFNB1* induction upon virus infection in fibroblasts (16).

The IFN-B adaptive immune response may be induced by various therapies, and in particular by radiotherapy and antiviral therapies.

It has notably been established that radiotherapy favors an anti-tumor immune response through a pathway involving IFN-B (17-21). Used for both palliative or curative purposes, radiotherapy induces tumor cell death along with an increase in serum IFN-B, resulting in an anti-tumor specific immune response. However, radiotherapy is not effective in all patients. It has been suggested that this inefficacy is associated with the absence of an increase in serum IFN-B (21). However, serum IFN-B levels can only be evaluated after administration of treatment.

Thus, there remains a need for a novel method capable of predicting the ability of a patient to produce an IFN-B dependent adaptive immune response (i.e. in response to a given therapy that is known to induce an IFN-B dependent adaptive immune response). Such a method would advantageously permit a personalized approach to therapy, in which treatment options are adapted according to the expected patient immune response.

In particular, there remains a need for a novel method of screening a subject diagnosed with cancer for responsiveness to radiotherapy. Such methods should be quick and easy to perform, and be available at low cost.

### Detailed description of the invention

In this context, the inventors have surprisingly found that a particular SNP, namely rs12553564, and three other SNPs in high linkage disequilibrium with said rs12553564 SNP (said three other SNPs being rs12551341, rs2275888, and rs10811449) are each associated with a modulation of the IFN-B dependent adaptive immune response. Without being limited by theory, it is thought that the modulation of the IFN-B dependent adaptive immune response results from the disruption of a conserved C/EBP-B binding site by the rs12553564 minor allele (G nucleotide), preventing C/EBP-B binding and inhibiting LPS-inducible enhancer activity which would otherwise increase *IFNB1* gene expression, and thus IFN-B production. The presence of a minor allele in any of the three other SNPs reveals that the minor allele is present in the rs12553564 SNP.

According to a first aspect, the present invention therefore relates to an *in vitro* method for predicting the efficiency of a treatment stimulating an IFN-B dependent adaptive immune response, said method comprising a step of detecting the rs12553564 single nucleotide polymorphism (SNP), or an SNP in high linkage disequilibrium with same, said SNP being selected from rs12551341, rs2275888, and rs10811449, in a biological sample of a subject in need thereof. This method provides rapid and simple means of determining if a subject will respond to a treatment which requires an IFN-B dependent adaptive immune response for efficacy. Given the ease of detecting specific nucleic acid sequences, the method may be performed at a very low cost.

IFN-B is a proinflammatory cytokine which has potent antiviral and immuno-modulatory activities. It is known to be a stimulator of the differentiation and activity of dendritic cells (DCs) (22). IFNB may enhance cell-surface expression of MHC molecules and co-stimulatory molecules, such as CD80 and CD86, which are associated with an increased ability to stimulate T cells. IFNβ may also promote the ability of DCs to cross-present antigens during viral infections and/or promote the migration of DCs to lymph nodes thus promoting T cell activation (2). In particular, radiation-mediated anti-tumor immunity is known to depend on IFN-B, which enhances the ability of dendritic cells (DCs) to cross-prime CD8⁺ T cells (17). As a non-limiting example, IFN-B may modulate the adaptive immune response via one or more of these mechanisms.

As well-known in the art, an "adaptive immune response" is antigen-specific and requires the recognition of specific self or non-self antigens during a process called antigen presentation. Antigen specificity allows for the generation of responses that are tailored to specific pathogens or pathogen infected cells or tumor cells. The ability to mount these tailored responses is maintained in the body by so-called "memory cells". Should a pathogen infect the body more than once, these specific memory cells are used to quickly eliminate said pathogen. The adaptive immune system thus allows for a stronger immune response as well as for an immunological memory, where each pathogen or tumor cell is remembered by one or more signature antigens.

The major components of the adaptive immune system in vertebrates predominantly include lymphocytes on the cellular level and antibodies on the molecular level. Lymphocytes as cellular components of the adaptive immune system include B cells and T cells which are derived from hematopoietic stem cells in the bone marrow. B cells are involved in the humoral response, whereas T cells are involved in cell mediated immune response. Both B cells and T cells carry receptor molecules that recognize specific targets.

Thus, the term "IFN-B dependent adaptive immune response" as used herein refers to the adaptive immune response insofar as it is induced by IFN-B (e.g. by enhancing the ability of dendritic cells (DCs) to cross-prime CD8⁺ T cells, or locally recruiting immune-competent cells).

The term "treatment" as used herein refers to any treatment comprising a drug or therapy which induces an IFN-B dependent adaptive immune response in a subject. As a non-limiting example, said treatment may be a radiotherapy, an anti-cancer chemotherapy drug, or an anti-microbial drug, such as an anti-viral drug.

The term "radiotherapy" as used herein refers to any therapy that treats a disease by delivery of energy through electromagnetic radiation. "Radiation" as used herein includes the range from gamma radiation to radiowaves and includes x-ray, ultraviolet, visible, infrared, microwave, and radiowave energies. X-ray radiation generally refers to photons with wavelengths below about 10 nm down to about 0.01 nm. Gamma rays refer to electromagnetic waves with wavelengths below about 0.01 nm. Ultraviolet radiation refers to photons with wavelengths from about 10 nm to about 400 nm. Visible radiation refers to photons with wavelengths from about 400 nm to about 700 nm. Photons with wavelengths above 700 nm are generally in the infrared radiation regions. Within the x-ray regime of electromagnetic radiation, low energy x-rays can be referred to as orthovoltage. While the exact photon energies included within the definition of orthovoltage varies, for the disclosure herein, orthovoltage refers at least to x-ray photons with energies from about 20 keV to about 500 keV.

The amount of radiation used in photon radiation therapy is measured in gray (Gy), which is equal to 1 joule per kilogram, which generally also equals 100 rad. The amount of radiation used in photon radiation therapy varies depending on the type of disease being treated (e.g. cancer) and its stage of progression. As a non-limiting example, for curative treatment, the typical dose for a solid epithelial tumor ranges from 60 to 80 Gy, while lymphomas are treated with 20 to 40 Gy.

Radiotherapy may be administered externally (e.g. via external beam radiotherapy) or internally via treatment with a radioactive compound, such as a radioisotope or radionuclide (e.g. iodine-131, phosphorus-32, radium-223, strontium-89, samarium-153), or implant (e.g. brachytherapy).

The "anti-cancer chemotherapy drug" may be any drug used in cancer treatment having a cytotoxic effect and which further induces IFN-B expression. As a non-limiting example, the anti-cancer chemotherapy drug may be topotecan, etoposide, cisplatin, paclitaxel or vinblastine (23).

The "anti-microbial drug" may be any drug used in the treatment of infection due to a bacteria, fungus, or virus, which induces IFN-B expression. The anti-microbial drug may be an anti-bacterial drug, an anti-fungal drug, or an anti-viral drug.

Thus, according to a preferred embodiment, the treatment on which the predictive method of the invention is applied is selected from radiotherapy, an anti-cancer chemotherapy drug, or an anti-microbial drug.

The expression "single nucleotide polymorphism" herein refers to a variation of DNA sequence at a single nucleotide position in the genome of a subject, e.g. a human being. An SNP is therefore a stable variation of the DNA sequence at the level of a single nucleotide base. An SNP according to the invention defines a single locus. It is expressed according to the reference number (rs) assigned by the NCBI database (https://www.ncbi.nlm.nih.gov/snp). The SNP may be polymorphic: the same individual may carry two copies of the same SNP (homozygote) or two different SNPs (heterozygote) at the same locus. An SNP, and thus the corresponding allele, can be located within a coding region of a gene, in the non-coding region of a gene, or in the intergenic region between genes.

The "rs12553564" SNP more particularly refers to the SNP which is located in humans on chromosome 9 at position 21,017,241 (human genome version GRCh38.p12 of Dec. 21, 2017). The locus of said rs12553564 SNP is diploid (i.e. it can have two different alleles). The term "allele" refers to a variant in the nucleotide sequence of a locus. More precisely, the nucleotide base at the locus of the rs12553564 SNP may be either the A allele or the G allele.

The "rs12551341" SNP more particularly refers to the SNP which is located in humans on chromosome 9 at position 21,014,629 (human genome version GRCh38.p12 of Dec. 21, 2017). The locus of said rs12551341 SNP is diploid (i.e. it can have two different alleles). The term "allele" refers to a variant in the nucleotide sequence of a locus. More precisely, the nucleotide base at the locus of the rs12551341 SNP may be either the T allele or the C allele.

The, "rs2275888" more particularly refers to the SNP which is located in humans on chromosome 9 at position 21,017,885 (human genome version GRCh38.p12 of Dec. 21, 2017). The locus of said rs2275888SNP is diploid (i.e. it can have two different alleles). The term "allele" refers to a variant in the nucleotide sequence of a locus. More precisely, the nucleotide base at the locus of the rs2275888 SNP may be either the T allele or the C allele.

The "rs10811449" more particularly refers to the SNP which is located in humans on chromosome 9 at position 21,009,192 (human genome version GRCh38.p12 of Dec. 21, 2017). The locus of said rs10811449 SNP is diploid (i.e. it can have two different alleles). The term "allele" refers to a variant in the nucleotide sequence of a locus. More precisely, the nucleotide base at the locus of the rs10811449 SNP may be either the G allele or the A allele.

The alleles of the rs12553564 SNP may be present in different proportions in a given population. The alleles of the rs12553564 SNP may notably be in linkage disequilibrium with other SNPs, such one or more of those indicated in Table 2. The term "linkage disequilibrium" or "LD" as used herein refers to the non-random association between two or more alleles at two or more loci such that certain combinations of alleles are more likely to occur together on a chromosome than other combinations of alleles that would be expected from a random formation of haplotypes from alleles based on their frequencies (e.g. the association of the G allele at the rs12553564 SNP with the C allele at the rs12551341 SNP, the C allele at the rs2275888 SNP and/or the A allele at the rs10811449 SNP).

In the context of the invention, the inventors have shown that the minor allele of the rs12553564 SNP is in high linkage disequilibrium with the minor allele of the rs12551341, rs2275888, and rs10811449 SNPs (Table 2).

They have also shown that the minor allele of the rs12553564 SNP is in linkage disequilibrium with other minor alleles, for example with the minor alleles of an SNP selected from rs58788481, rs7871739, rs6475498, rs7033035, rs12115505, rs7868923, rs35641645, rs9777591, rs2298260, rs10964800, rs71496869, rs2039389, and rs10964817 (see also Table 2).

In the context of the present invention, two or more alleles have a "high linkage disequilibrium" when the r² value is equal or superior to 0.9 (see e.g. the r² values as provided in Table 2). Thus, the rs12553564 SNP notably has a high LD with the rs2275888, rs12551341, and rs10811449 SNPs respectively. It should nevertheless be noted that LD may notably vary among population groups. Thus, the rs12553564 SNP may be in high LD with one or more SNPs selected from rs58788481, rs7871739, rs6475498, rs7033035, rs12115505, rs7868923, rs35641645, rs9777591, rs2298260, rs10964800, rs71496869, rs2039389, and rs10964817 in certain populations.

More specifically, the G allele of the rs12553564 SNP may be in high LD with one or more of the following: the G allele at the rs58788481 SNP, the A allele at the rs7871739 SNP, the C allele at the rs6475498 SNP, the G allele at the rs7033035 SNP, the C allele at the rs12115505, the T allele at the rs7868923 SNP, the A allele at the rs35641645 SNP, the A allele at the rs9777591 SNP, the C allele at the rs2298260 SNP, the T allele at the rs10964800 SNP, the G allele at the rs71496869 SNP, the G allele at the rs2039389 SNP, and the T allele at the rs10964817 SNP, in certain populations. In a preferred embodiment, the rs12553564 SNP is in high LD with one or more SNPs selected from rs58788481, rs7871739, rs6475498, rs7033035, rs12115505, rs7868923, rs35641645, rs9777591, rs2298260, rs10964800, rs71496869, rs2039389, and rs10964817. Therefore, any of these SNPs could be in fact be used in the methods of the invention, in certain populations. More precisely, the presence of the minor G allele at the rs58788481 SNP, the minor A allele at the rs7871739 SNP, the minor C allele at the rs6475498 SNP, the minor G allele at the rs7033035 SNP, the minor C allele at the rs12115505, the minor T allele at the rs7868923 SNP, the minor A allele at the rs35641645 SNP, the minor A allele at the rs9777591 SNP, the minor C allele at the rs2298260 SNP, the minor T allele at the rs10964800 SNP, the minor G allele at the rs71496869 SNP, the minor G allele at the rs2039389 SNP, and the minor T allele at the rs10964817 SNP, is likely to be predictive of the response to a treatment stimulating an IFN-B dependent adaptive immune response, in certain populations.

As shown in the Examples below, the rs12553564, rs2275888, rs12551341, and rs10811449 SNPs are more particularly present in non-coding regions of the *HACD4* gene. The rs12553564 SNP notably influences the level of expression of the *IFN1B* gene. As the rs12553564 SNP present in the non-coding region, as well as the rs2275888, rs12551341, and rs10811449 SNPs which are in high LD with the rs12553564 SNP, are linked to quantitative defects in the corresponding IFN-β protein, they will thus be referred to hereafter as "expression quantitative trait loci (eQTL)."

In the context of the present invention, the minor allele present at the rs12553564 SNP is a G allele. Thus, the nucleotide base at the rs12553564 locus may be either the major A allele or the minor G allele. In humans, each copy of the locus may be the same or different. Thus, the rs12553564 locus may be a homozygous A/A allele, a heterozygous A/G allele, or a homozygous G/G allele.

In the present context, the minor allele present at the rs12551341 SNP is a C allele. Thus, the nucleotide base at the rs12551341 locus may be either the major T allele or the minor C allele. In humans, each copy of the locus may be the same or different. Thus, the rs12551341 locus may be a homozygous T/T allele, a heterozygous T/C allele, or a homozygous C/C allele.

In the present context, the minor allele present at the rs2275888 SNP is a C allele. Thus, the nucleotide base at the rs2275888 locus may be either the major T allele or the minor C allele. In humans, each copy of the locus may be the same or different. Thus, the rs2275888 locus may be a homozygous T/T allele, a heterozygous T/C allele, or a homozygous C/C allele.

In the present context, the minor allele present at the rs10811449 SNP is an A allele. Thus, the nucleotide base at the rs10811449 locus may be either the major A allele or the minor G allele. In humans, each copy of the locus may be the same or different. Thus, the rs10811449 locus may be a homozygous G/G allele, a heterozygous G/A allele, or a homozygous A/A allele.

As shown in the EXAMPLES below, the presence of homozygous G alleles at the rs12553564 SNP in a subject indicates that a treatment stimulating an IFN-B dependent adaptive immune response will be less efficient in said subject. Similarly, minor alleles of an SNP in high LD with the rs12553564 SNP (i.e. homozygous C alleles of the rs12551341 or rs2275888 SNPs or homozygous A alleles of the rs10811449 SNP) in a subject indicates that a treatment stimulating an IFN-B dependent adaptive immune response will be less efficient in said subject. The same is true when homozygous minor alleles are found in the other SNPs disclosed in Table 2, in certain populations.

Thus, in the context of personalized medicine, when homozygous G alleles (or homozygous C alleles of the rs12551341 or rs2275888 SNPs or homozygous A alleles of the rs10811449 SNP or homozygous minor alleles of the SNPs disclosed in Table 2) are detected, a treatment other than a treatment stimulating an IFN-B dependent adaptive immune response, or a treatment stimulating an IFN-B dependent adaptive immune response but having an increased dosage, may advantageously be selected.

Alternatively, the presence of homozygous A alleles of the rs12553564 SNP or homozygous major alleles of an SNP in high LD with the rs12553564 SNP (e.g. homozygous T alleles of the rs12551341 or rs2275888 SNPs or homozygous G alleles of the rs10811449 SNP, or homozygous major alleles of the other SNPs disclosed in Table 2) in a subject indicates that a treatment stimulating an IFN-B dependent adaptive immune response is likely to be efficient in said subject.

The presence of a heterozygous A/G allele of the rs12553564 SNP or heterologous alleles of an SNP in high LD with the rs12553564 SNP (e.g. T/C alleles of the rs12551341 or rs2275888 SNPs or G/A alleles of the rs10811449 SNP) may indicate that a treatment stimulating an IFN-B dependent adaptive immune response is likely to be efficient in said subject. Alternatively, the presence of a heterozygous A/G allele of the rs12553564 SNP or heterologous alleles of an SNP in high LD with the rs12553564 SNP (e.g. T/C alleles of the rs12551341 or rs2275888 SNPs or G/A alleles of the rs10811449 SNP) may indicate that a treatment stimulating an IFN-B dependent adaptive immune response will be less efficient in said subject. Indeed, the heterozygous response may notably vary according to the cancer type. Preferably, a heterozygous A/G allele of the rs12553564 SNP or heterologous alleles of an SNP in high LD with the rs12553564 SNP (e.g. T/C alleles of the rs12551341 or rs2275888 SNPs or G/A alleles of the rs10811449 SNP) indicates that a treatment stimulating an IFN-B dependent adaptive immune response is likely to be efficient in said subject. Alternatively, a heterozygous A/G allele of the rs12553564 SNP or heterologous alleles of an SNP in high LD with the rs12553564 SNP (e.g. T/C alleles of the rs12551341 or rs2275888 SNPs or G/A alleles of the rs10811449 SNP) preferably indicates that a treatment stimulating an IFN-B dependent adaptive immune response will be less efficient in said subject.

For the purposes of the present invention, the term "subject" herein means a mammal, preferably a human, irrespective of age. Thus, the subject may be for example an adult or a child. "Adult" means an individual who is at least 16 years of age. "Child" refers to an individual whose age is less than 16 years of age, particularly infants from birth to 1 year of age, and children from 1 to 15 years of age.

Preferably, said subject is suffering from a disease that is sensitive to an IFN-β-mediated adaptive immune response, such as a cancer or a microbial infection, such as a viral infection, or an auto-immune disease. In one embodiment, the method of the invention is particularly useful for subjects suffering from specific cancers, such as breast cancer, colorectal cancer, bladder cancer, liver cancer, pancreatic cancer, lung cancer, cervical cancer, thyroid cancer, leukemia (e.g. childhood acute lymphoblastic leukemia), skin cancer (e.g. melanoma, basal cell carcinoma), prostate cancer, stomach cancer, or a cancer of the head or neck (e.g. throat cancer, oral cancer), that may be subject to treatment with radiotherapy.

Thus, according to a preferred embodiment, the subject of the invention has a cancer, preferably a breast cancer, colorectal cancer, bladder cancer, liver cancer, pancreatic cancer, lung cancer, cervical cancer, thyroid cancer, leukemia, skin cancer, prostate cancer, stomach cancer, or a cancer of the head or neck.

The terms *"in vitro"* and *"ex vivo"* as used herein are equivalent and refer to methods that are conducted using biological components (e.g. tissues, cells, biological fluids) that have been isolated from their usual host organism (e.g. an animal or human). Such isolated cells or fluids can be directly used in the methods of the invention, without further processing. Alternatively, isolated cells may be purified and/or cultured before being used in the methods of the invention. These methods can be for example reduced to practice in laboratory materials such as tubes, flasks, wells, eppendorfs, etc. In contrast, the term *"in vivo"* refers to methods that are conducted on whole living organisms.

The expression "biological sample" as used herein refers to any sample comprising nucleic acids, obtained from a human subject. A sample may comprise tissues and/or biological fluids. Such samples can be obtained *in vitro, ex vivo* or *in vivo.*

As a non-limiting example, the biological sample may be selected from tissues, organs, cells, or any isolated fraction of a human subject. The biological sample may also be selected from biological fluids including but not limited to blood, plasma, lymph, saliva, urine, stool, tears, sweat, sperm, or cerebrospinal, synovial, pleural, peritoneal, or pericardial fluid, as well as any fraction thereof. In a preferred embodiment, said biological sample is a blood, plasma, lymph, or saliva sample of said subject, or bone marrow or spleen or skin biopsies, or any other cells.

In a particularly preferred embodiment, the biological sample is a biological fluid, preferably a blood, plasma, lymph or saliva sample.

The sample may also be pre-processed to preserve the integrity of the nucleic acids and/or to make them more accessible for further analysis. For example, the sample may be treated with anti-nucleases. The sample may also undergo lysis steps (e.g. chemical, mechanical, or enzymatic lysis), centrifugation, purification, etc. to facilitate access to nucleic acids and/or to concentrate them.

Said sample can be obtained by any technique known in the prior art. Said blood or plasma sample may be obtained by a completely harmless blood collection from the subject and thus advantageously allows for a non-invasive detection. The blood sample used in the method of the invention is preferably depleted of most, if not all, erythrocytes, using common red blood cell lysis procedures. Preferably, peripheral blood mononuclear cells are prepared from the blood sample. Said saliva sample may be obtained with a simple mouth swab or using the passive drool technique.

The term "detection" as used herein refers to any means allowing for the identification of the rs12553564 SNP or of an SNP in high LD with the rs12553564 SNP (e.g. the rs12551341, rs2275888, or rs10811449 SNP, or any other of those disclosed in Table 2). As a non-limiting example, detection of the SNP may be performed by allelic discrimination. The term "allelic discrimination" is used herein in a non-limiting manner, and comprises methods of hybridization, nucleotide incorporation, oligonucleotide ligation, invasive cleavage, enzymatic digestion, or sequencing, such that it is possible to determine the allele(s) present at the rs12553564 locus or at the locus of an SNP in high LD with the rs12553564 SNP (e.g. the rs12551341, rs2275888, or rs10811449 SNP or any other of those disclosed in Table 2).

The term "hybridization" as used herein refers to the formation of a specific complex between two single-stranded polynucleotide sequences due to complementary base pairing. "Specific complex formation" refers to the formation of a complex that is dependent on the precise sequence at the SNP locus. During hybridization, the presence of a mismatch error at the level of the base of interest destabilizes the interaction between the sequence of interest and the complementary sequence. This destabilization can be detected. Preferably, the destabilization of the interaction prevents hybridization. As a non-limiting example, hybridization may be performed during PCR. Hybridization may also be performed following a step of amplification of the nucleic acid. Preferably, hybridization takes place between a sequence of interest, comprising an SNP, and an oligonucleotide (e.g., a probe or primer).

The term "primer" as used herein refers to an isolated nucleic acid molecule that can specifically hybridize or anneal to a 5' or 3' region of a target genomic region (plus and minus strands, respectively, or vice-versa). In general, primers are from about 10 to 30 nucleotides in length and anneal at both extremities of a region that is about 50 to 1000 nucleotides in length, more preferably about 50 to 500 nucleotides in length, more preferably about 50 to 200 nucleotides in length. Under appropriate conditions and with appropriate reagents, such primers permit the amplification of a nucleic acid molecule comprising the target nucleotide sequence (i.e. a nucleotide sequence comprising the SNP locus) flanked by the primers. As they are used in pairs, they are often referred to as a "primer pair" or "primer set".

The term "probe" refers to a labeled oligonucleotide that specifically hybridizes with a nucleic acid molecule having a particular allele at the SNP locus. The interaction of the probe with a particular allele at the SNP locus can then be detected. As a non-limiting example, a probe may be coupled to a fluorescent, luminescent, radioactive, chemical, enzymatic, or electrical marker. The probe may comprise one or more non-natural nucleotides, e.g., a peptide nucleic acid (PNA), a peptide nucleic acid having a phosphate group (PHONA), a bridged nucleic acid or locked nucleic acid (BNA or LNA), and a morpholino nucleic acid. Non-natural nucleotides also include chemically modified nucleic acids or nucleic acid analogs such as methylphosphonate-type DNA or RNA, phosphorothioate-type DNA or RNA, phosphoramidate-type DNA or RNA, and 2'-O-methyl-type DNA or RNA. The length of a probe can be between 8 and 50 nucleotides. Preferably, the length of a probe is between 9 and 40 nucleotides. Preferably, the probe is a labeled probe of 8 to 50 contiguous nucleotides hybridizing with the rs12553564 SNP or an SNP in high LD with the rs12553564 SNP, preferably selected from rs12551341, rs2275888 and rs10811449 SNPs, or other SNPs disclosed in Table 2.

Preferably, the probe or the primer comprises a nucleotide base that is complementary to one of the alleles of the SNP. In one aspect, primer hybridization may be performed in the context of the multiplex allele-specific diagnostic method (MASDA) or a DNA chip. Alternatively, the SNP(s) may be detected via probe hybridization using a molecular beacon or a hydrolysis probe (e.g. Taqman), or specific dynamic allele-specific hybridization (DASH). Preferably, hybridization is performed on a solid support. Even more preferably, hybridization is performed on a DNA chip, which may be composed of oligonucleotides, DNA, or cDNA, or on a SNP chip.

As used herein, the expression "nucleotide incorporation" refers to the incorporation of a nucleotide that is complementary to the SNP locus. The nucleotide can be modified or labeled to facilitate detection. A nucleotide may be incorporated during the sequencing of said locus, during an amplification reaction, for example by PCR, or during primer extension. As a non-limiting example, a nucleotide may be labeled with a fluorescent, chemical, magnetic or radioactive molecule. As a non-limiting example, a nucleotide may be identified by measuring its mass. Preferably, the SNP(s) of the invention is detected by primer extension using nucleotide incorporation.

As used herein, the expression "oligonucleotide ligation" refers to a ligation between two oligonucleotides, one adjacent to the other, when their complementary base pairing is perfect at the ligation site. In a preferred embodiment, the SNP(s) of the invention is detected by oligonucleotide ligation.

As used herein, the expression "invasive cleavage" refers to the formation of a cleavage-sensitive structure when overlapping probes hybridize.

Oligonucleotide ligation and invasive cleavage methods do not require a prior amplification step.

As used herein, the expression "enzymatic digestion" refers to the digestion of a sequence by restriction enzymes that are dependent on the presence of a given SNP allele (e.g. A or G for the rs12553564 SNP). The restriction fragments can then be analyzed on a gel, for example by restriction fragment length polymorphism (RFLP). "Restriction fragments" refers to any fragment derived from an enzymatic digestion in which the enzyme cuts the double-stranded DNA at a specific sequence.

In a preferred embodiment, the SNP of the invention is detected by RFLP, by primer extension, by oligonucleotide ligation or by nuclease digestion.

According to another preferred embodiment, the SNP of the invention is detected by probe hybridization, amplification, sequencing, mass spectrometry, Southern blotting, or by any combination of these techniques.

In a preferred embodiment, allelic discrimination is performed by sequencing. "Sequencing" refers to a method for determining the sequence of a nucleic acid. A large number of sequencing methods are known in the art. For example, sequencing methods include Sanger dideoxy or chain terminated sequencing, whole genome sequencing, hybridization sequencing, pyrosequencing, capillary electrophoresis, cycle sequencing, sequencing. single base extension, solid phase sequencing, high throughput sequencing, massively parallel signature sequencing, nanopore sequencing, transmission electron microscopy sequencing, optical sequencing, mass spectrometry, 454 sequencing, labeled reversible terminator sequencing, "paired end" or "even mate" sequencing, exonuclease sequencing, ligation sequencing (e.g. according to SOLiD technology), short read sequencing, single molecule sequencing, chemical degradation sequencing, synthetic sequencing, mass parallel sequencing, real-time sequencing, semiconductor ion sequencing (e.g. Ion Torrent), multiplex sequencing of paired-end ditags (MS-PET), microfluidics, and combinations of these methods.

In the context of the invention, the sequencing should be performed on DNA, as the SNP(s) of the invention is present in a non-coding region. Optionally, said DNA may be randomly fragmented prior to sequencing. Sequencing of SNPs can be performed by any technique known in the art.

According to a preferred embodiment, the SNP(s) of the invention is detected by sequencing, more particularly by direct sequencing, ligation, synthesis, chain termination, single molecule real-time, semiconductor ion, microfluidics, mass parallel sequencing, or pyrosequencing.

One approach is to use a method allowing the quantitative genotyping of nucleic acids obtained from the biological sample with a high level of precision. According to a particular embodiment, this precision is obtained by analysis of a large number of nucleic acid molecules (e.g. millions or billions) without any prior amplification step, using protocols that rely on prior knowledge of target sequences (in this case, an SNP). In a preferred embodiment, the mass parallel sequencing method is used. As a non-limiting example, this method can be carried out using the "Illumina Genome Analyzer" platform (24), the Roche 454 platform (25), the ABI SOLiD platform (26), the Helicos single-molecule sequencing platform (27), the single-molecule sequencing in real time (28), Ion Torrent sequencing (29; WO 2010/008480), or nanopore sequencing (30).

Preferably, mass parallel sequencing is performed on a random subset of nucleic acid molecules in the biological sample.

Even more preferably, the method of the present invention is adapted to operate on an ABI PRISM^{®} 377 DNA sequencer, an ABI PRISM^{®} 310, 3100, 3100-Avant, 3730, or 3730x1 genetic analyzer, an Applied Biosystems SOLiD^{™} system (all from Applied Biosystems), a Genome Sequencer 20 system (Roche Applied Science), a HiSeq 2500, a HiSeq 2000, a Type IIx genomic analyzer, a MiSeq personal sequencer, a HiScanSQ (all from Illunima), the genetic analysis system including the *Single Molecule Sequencer,* the *Analysis Engine* and the *Sample Loader* (all from HeliScope), the Ion Proton^{™} or Ion PGM^{™} sequencer (both Ion Torrent).

Sequencing can also comprise methods based on polymerase chain reaction (PCR), such as quantitative PCR or emulsion PCR.

According to another preferred embodiment, the SNP(s) of the invention can also be detected by amplification. The amplification more particularly comprises isothermal methods as well as PCR methods.

Preferably, the SNP(s) of the invention is/are detected by PCR.

Preferably, the method provided herein further comprises, prior to detecting the rs12553564 SNP, or an SNP in high linkage disequilibrium with the rs12553564 SNP (e.g., selected from rs12551341, rs2275888, and rs10811449), the steps of:
a) isolating the nucleic acids from the biological sample, and
b) amplifying the nucleic acid.

As used herein, the term "nucleic acid" refers to any linear sequence of polynucleotides (e.g. of genomic DNA), such as oligonucleotides, primers, probes, amplicons, oligomeric fragments, etc. Preferably the nucleic acid is present in a biological sample from a human subject. It can be single stranded, double stranded, or a mixture of both forms. The nucleic acid may comprise coding and/or non-coding sequences. It may correspond to a fragment of an entire nucleic acid molecule. Preferably, the nucleic acid is DNA. Even more preferably, the nucleic acid is genomic DNA or a fragment thereof.

As used herein, the terms "nucleic acid isolation" and "isolating nucleic acid" refer to obtaining a sample comprising the nucleic acids of a human subject. Isolation may further comprise the "purification" of said nucleic acid. "Purification" refers to any process increasing the proportion of nucleic acid molecules vis-à-vis the other components of a sample, or isolating the nucleic acid molecules from other components of a sample. Purification may be partial or complete. It may comprise mechanical, enzymatic and/or chemical methods. For example, isolation may comprise a step of destabilizing a cell structure, e.g. by lysis. Isolation may also comprise a step of degrading other components, e.g. enzymatic degradation of proteins. Isolation may comprise a step of separating said nucleic acid from the other components by centrifugation, precipitation, binding to a solid support (e.g. to a silica membrane, by chromatography, to magnetic beads), organic extraction (e.g. by phenol-chloroform), etc.

As used herein, the term "amplification" refers to any process for increasing the amount of a nucleic acid molecule relative to its initial level. Amplification is dependent on the nucleic acid template and may be specific (e.g. using specific primers corresponding to exact sequences, by polymerase chain reaction (PCR)) or nonspecific (e.g. by multiple displacement amplification using hexamers). Methods for carrying out such amplification are well-known to the person skilled in the art.

More preferably, the amplification is performed by PCR with primers allowing for the amplification of a fragment of sequence SEQ ID NO: 33, 35, 37, or 39, said sequences of about 500 bp comprising the rs12553564 SNP, the rs12551341, the rs2275888, and the rs10811449 SNP respectively. This amplification is preferably performed by isothermal amplification of said sequence.

Preferably, the amplification is performed by PCR with primers of 12 to 30 contiguous nucleotides or by isothermal amplification of said SNP such that at least the nucleic acid of sequence SEQ ID NO: 34, 36, 38, or 40 is amplified. SEQ ID NO:34, 36, 38 and 40 correspond to nucleotide fragments of about 20 bp, containing the rs12553564 SNP, the rs12551341, the rs2275888, and the rs10811449 SNP respectively.

Preferably, said primers comprise 12 to 30 contiguous nucleotides of SEQ ID NO:33, 35, 37, or 39. Preferably, for amplifying the rs12553564 SNP, the primers have the sequences of SEQ ID NOs: 1 and 2. Preferably, for amplifying the rs12551341 SNP the primers have the sequences of SEQ ID NOs: 3 and 4.

According to another preferred embodiment, amplification of the nucleic acid comprising the SNP is performed by isothermal amplification. Preferably, the isothermal amplification consists of strand displacement amplification (SDA), helicase dependent amplification (HDA), loop mediated isothermal amplification (LAMP), nucleic acid sequence-based amplification (NASBA), rolling circle amplification (RCA), multiple displacement amplification (MDA) and recombinase polymerase amplification (RPA), exponential amplification reaction (EXPAR), isothermal and chimeric primer-initiated amplification of nucleic acids (ICAN), signal mediated amplification of RNA technology (SMART), nicking enzyme amplification reaction (NEAR)) and others (see, e.g., 31).

A kit for the detection of the rs12553564 SNP, or of an SNP in high linkage disequilibrium with the rs12553564 SNP (e.g., selected from rs12551341, rs2275888, and rs10811449 or other in Table 2), according to any of the methods described herein is further provided.

As used herein, the term "kit" refers to any system for delivering materials. In the context of reaction assays, it includes systems that allow the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials.

The kit may notably comprise primers for the amplification of a nucleic acid fragment comprising the rs12553564 SNP, or an SNP in high linkage disequilibrium with the rs12553564 SNP (e.g., selected from rs12551341, rs2275888, and rs10811449 or other SNP disclosed in Table 2). Said fragment comprises for example the nucleic acid sequence of SEQ ID NO: 34, 36, 38, or 40 or SEQ ID NO:33, 35, 37, or 39. The kit may further comprise a polymerase enzyme for nucleic acid amplification. The kit may comprise one or more probes, restriction enzymes, nucleases, and/or primers for the detection of the rs12553564 SNP, or of an SNP in high linkage disequilibrium with the rs12553564 SNP (said SNP being selected from rs12551341, rs2275888, and rs10811449, or other SNPs in Table 2) as provided herein. The kit may comprise any appropriate buffers or written instructions.

Preferably, the kit comprises at least one probe that is complementary to the A allele of the SNP and/or at least one probe that is complementary to the G allele of the rs12553564 SNP. Preferably, the kit comprises at least one probe that is complementary to the T allele of the SNP and/or at least one probe that is complementary to the C allele of the rs12551341 SNP. Preferably, the kit comprises at least one probe that is complementary to the T allele of the SNP and/or at least one probe that is complementary to the C allele of the rs2275888 SNP. Preferably, the kit comprises at least one probe that is complementary to the G allele of the SNP and/or at least one probe that is complementary to the A allele of the rs10811449 SNP. Preferably, the kit comprises at least one probe that is complementary to the major allele of the SNP and/or at least one probe that is complementary to the minor allele of another SNP disclosed in Table 2.

In other words, the kit preferably allows for the detection of the presence of the homozygous G alleles of the rs12553564 SNP, the homozygous C alleles of the rs12551341 SNP, the homozygous C alleles of the rs2275888 SNP, or the homozygous A alleles of the rs10811449 SNP or for any minor allele of another SNP disclosed in Table 2.

According to a further aspect, the present invention relates to the use of a kit containing the means to detect the rs12553564 SNP, or an SNP in high linkage disequilibrium with the rs12553564 SNP (e.g. selected from rs12551341, rs2275888, and rs10811449 or any other SNP disclosed in Table 2), in a nucleic acid, for predicting the efficiency of a treatment stimulating an IFN-B dependent adaptive immune response in a subject in need thereof, wherein said subject is preferably diagnosed with cancer.

Preferably, said kit contains reagents for detecting the rs12553564 SNP within SEQ ID NO: 33, the rs12551341 SNP within SEQ ID NO: 35, the rs2275888 SNP within SEQ ID NO: 37, or the rs10811449 within SEQ ID NO: 39, preferably said reagents comprise primers and/or a probe. Preferably, said reagents comprise the primers of SEQ ID NOs: 1 and 2, for amplifying a nucleic acid fragment comprising rs12553564.

According to a further aspect, the present invention relates to an *in vitro* method of screening a subject diagnosed with cancer for responsiveness to radiotherapy comprising:
a) genotyping the rs12553564 SNP, or an SNP in high linkage disequilibrium with the rs12553564 SNP (e.g., selected from rs12551341, rs2275888, and rs10811449 or any other SNP disclosed in Table 2), in a biological sample of the subject, and
b) determining the responsiveness of the subject to radiotherapy.

In a particular embodiment, the presence of a homozygous G allele of the rs12553564 SNP, a homozygous C allele of the rs12551341 SNP or of the rs2275888 SNP, a homozygous A allele of the rs10811449 SNP, or a minor homozygous allele in other SNPs disclosed in Table 2, is indicative of non-responsiveness to radiotherapy. Alternatively, the presence of a homozygous A allele or a heterozygous A/G allele of the rs12553564 SNP (or the presence of a homozygous major allele or a heterozygous allele of rs12551341, rs2275888, or rs10811449 or other SNPs disclosed in Table 2) will be indicative of a significant responsiveness to radiotherapy.

Indeed, as shown in the Examples below (see point 2.3), the inventors have surprisingly found that the presence of homozygous G alleles of the rs12553564 SNP is correlated to a reduced responsiveness to radiotherapy (16.7% responsiveness as compared to more than 42% responsiveness in subjects having homozygous A alleles or heterozygous A/G alleles). In cases where the presence of a homozygous G allele of the rs12553564 SNP, a homozygous C allele of the rs12551341 SNP or of the rs2275888 SNP, or a homozygous A allele of the rs10811449 SNP, is detected, the method may notably further comprise: c) selecting a treatment regimen comprising exogenous IFN-B, exogenous IFN-α, and/or a checkpoint inhibitor drug, and/or increasing the radiotherapy dosage.

In cases where the presence of a homozygous G allele of the rs12553564 SNP, a homozygous C allele of the rs12551341 SNP or of the rs2275888 SNP, or a homozygous A allele of the rs10811449 SNP (or a homozygous minor allele of another SNP disclosed in Table 2) is detected, the method advantageously further comprises:
c) selecting a treatment regimen comprising exogenous IFN-B, exogenous IFN-α, and/or a checkpoint inhibitor drug.

A "checkpoint inhibitor drug" as used herein refers to a drug that blocks immune system checkpoint proteins from binding to their partner proteins on the surface of T-cells, which would otherwise lead to a reduction in the immune response to a stimulus. As a non-limiting example, the checkpoint inhibitor drug is an anti-CTLA-4 such as ipilimumab, an anti-PD1 such as nivolumab, pembrolizumab, or spartalizumab or an anti-PD-L1 such as atezolizumab.

In cases where the presence of a homozygous G allele of the rs12553564 SNP, a homozygous C allele of the rs12551341 SNP or of the rs2275888 SNP, or a homozygous A allele of the rs10811449 SNP (or a homozygous minor allele of any other SNP disclosed in Table 2), is detected, the method advantageously further comprises:
c) selecting a treatment regimen which does not comprise a therapy that stimulates an IFN-B dependent adaptive immune response, preferably which does not comprise radiotherapy.

According to a further aspect, a method for *in vitro* assessing whether a radiotherapy is appropriate for a subject diagnosed with cancer, comprising a step of detecting the rs12553564 SNP, or an SNP in high linkage disequilibrium with the rs12553564 SNP (e.g., selected from rs12551341, rs2275888, and rs10811449 or other SNPs disclosed in Table 2), in a biological sample of the subject, is provided.

According to a further aspect, an *in vitro* screening method for selecting a subject suffering from cancer for a radiotherapy treatment, comprising a step of detecting the rs12553564 SNP, or an SNP in high linkage disequilibrium with the rs12553564 SNP (e.g., selected from rs12551341, rs2275888, and rs10811449 or other SNPs disclosed in Table 2), in a biological sample of the subject, is provided.

The present invention also relates to an *in vitro* method for adapting a treatment of a human subject suffering from cancer, comprising:
a) detecting the rs12553564 SNP, or an SNP in high linkage disequilibrium with the rs12553564 SNP (e.g., selected from rs12551341, rs2275888, and rs10811449 or other SNPs disclosed in Table 2), in a biological sample of the subject, and
b) adapting the treatment of said subject.

Preferably, the adaptation of the treatment comprises a treatment with exogenous INF-B, IFN-α, and/or a checkpoint inhibitor drug when homozygous G alleles of the rs12553564 SNP, homozygous C alleles of the rs12551341 SNP or of the rs2275888 SNP, or homozygous A alleles of the rs10811449 SNP (or a homozygous minor allele of another SNP disclosed in Table 2), are detected. Preferably, the adaptation of the treatment comprises the exclusion of radiotherapy as a treatment option when homozygous G alleles of the rs12553564 SNP, homozygous C alleles of the rs12551341 SNP or of the rs2275888 SNP, or homozygous A alleles of the rs10811449 SNP (or a homozygous minor allele of another SNP disclosed in Table 2), are detected. Alternatively, the adaptation of the treatment comprises an increase in radiotherapy dose. Preferably, the adaptation of the treatment comprises the administration of a medicament stimulating an IFN-β dependent adaptive immune response, more preferably the administration of exogenous IFN-B, exogenous IFN-α, and/or a checkpoint inhibitor drug, when homozygous G alleles are detected for the rs12553564 SNP, homozygous C alleles are detected for the rs12551341 SNP or for the rs2275888 SNP, or homozygous A alleles are detected for the rs10811449 SNP (or when other homozygous minor alleles are detected for the other SNPs disclosed in Table 2).

According to a further aspect, the use of primers and/or probes that can specifically amplify or hybridize the genomic region of SEQ ID NO:33, 35, 37, or 39 containing rs12553564, rs12551341, rs2275888, and rs10811449 respectively, or a fragment thereof (e.g. comprising SEQ ID NO: 34, 36, 38 or 40), for *in vitro* predicting the efficiency of a treatment stimulating an IFN-B dependent adaptive immune response is provided herein.

The present invention also relates to the use of rs12553564, or an SNP in high linkage disequilibrium with the rs12553564 SNP (e.g., selected from rs12551341, rs2275888, and rs10811449 or other SNPs disclosed in Table 2), as a prognostic marker of responsiveness to a treatment stimulating an IFN-B dependent adaptive immune response.

The present invention also relates to an *in vitro* method for predicting the severity of a disease inducing an IFN-B dependent adaptive immune response in a subject, comprising a step of detecting the rs12553564 SNP, or an SNP in high linkage disequilibrium with the rs12553564 SNP (e.g., selected from rs12551341, rs2275888, and rs10811449 or other SNPs disclosed in Table 2), in a biological sample of said subject. Preferably said disease is a viral infection, more preferably SARS-CoV-2. Said subject may or may not have been diagnosed with the disease. In cases where said subject is diagnosed with the disease, the method preferably further comprises a step of adapting the treatment regimen of said subject. More preferably, the presence of homozygous G alleles of the rs12553564 SNP, or of homozygous C alleles of the rs12551341 SNP, homozygous C alleles of the rs2275888 SNP, or homozygous A alleles of the rs10811449 SNP, or of a minor allele in another SNP disclosed in Table 2, indicates that the disease will be more severe. As a non-limiting example, in the case of viral infection, the dosage of anti-viral may be increased.

Said SNP may be detected by any of the techniques provided herein. Said SNP may be detected in any biological sample as provided herein.

The present invention further relates to the use of the rs12553564 SNP, or of an SNP in high linkage disequilibrium with the rs12553564 SNP (e.g., selected from rs12551341, rs2275888, and rs10811449, or other SNPs disclosed in Table 2), as a prognostic marker of disease severity in a subject, preferably a disease inducing an IFN-B dependent adaptive immune response in a subject. Preferably said disease is a viral infection, more preferably SARS-CoV-2.

### Figure legends

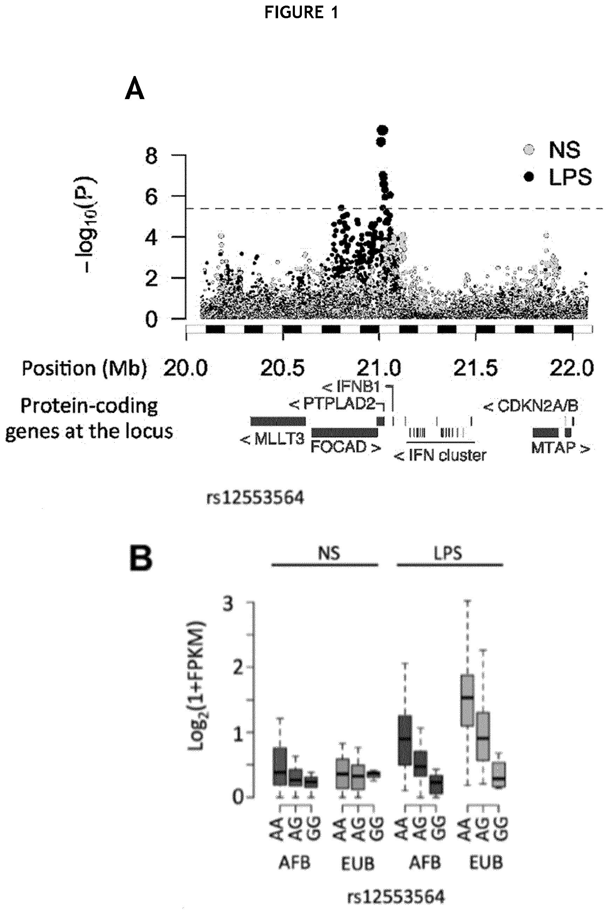

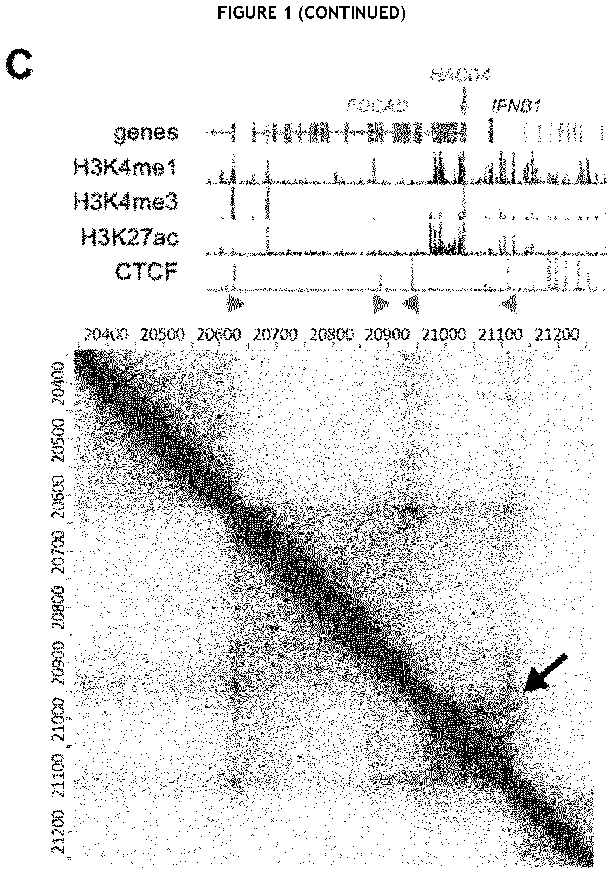

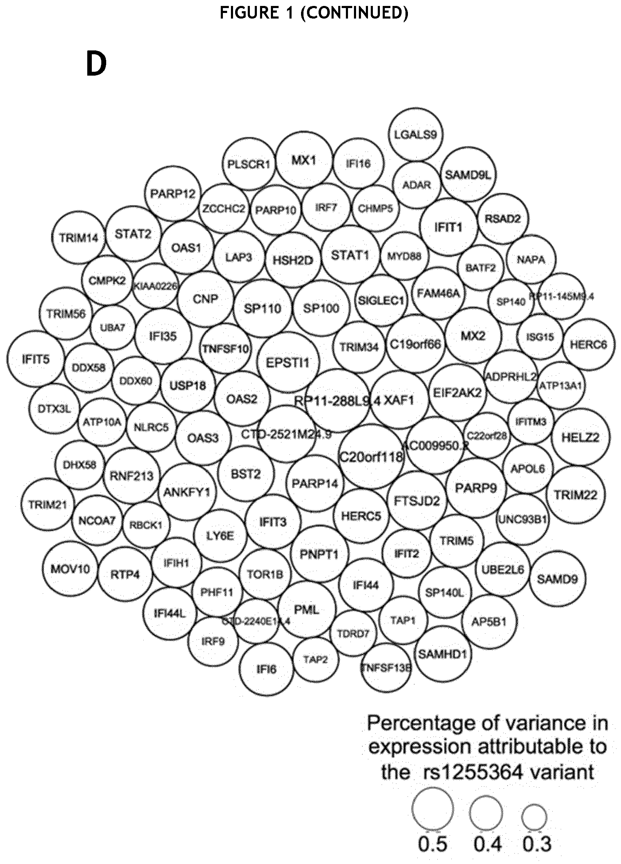

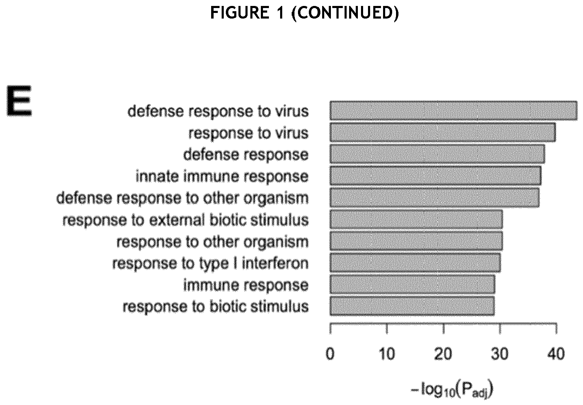

**Figure 1****. A genetic variant is associated with a decreased interferon response in myeloid cells.**
   (A) Association of SNPs within 1Mb of *IFNB1* with *IFNB1* expression in non-stimulated (grey) and LPS-stimulated (pink) monocytes. Dotted line indicates the 1% Family wise error rate obtained by permutation. Significant SNPs are highlighted in red. *(B) IFNB1* expression for each genotype of rs12553564 in 2 populations (AFB: African ancestry from Belgium, EUB: European ancestry from Belgium), in non-stimulated (grey) and LPS-stimulated (red) monocytes. (C) Hi-C analysis of the FIRE region in THP-1 cells. Genes are indicated on top, with *IFNB1* in red. Histone marks of promoters (H3K4me3) and enhancers (H3K4me1 and H3K27ac) are aligned, as well as oriented CTCF peaks. The black arrow indicates the loop containing *IFNB1* and rs12553564. (D) Top 100 genes most strongly associated to rs12553564 upon LPS stimulation. Each gene is represented by a circle colored according to the fold change in expression between both alleles of the variant. Size reflects the percentage of variance in gene expression accounted for by the variant. (*E*) Functional enrichments of IFNB1 trans -regulated genes. -Log₁₀(adjusted p-values) are reported for the top 10 most enriched GO categories.

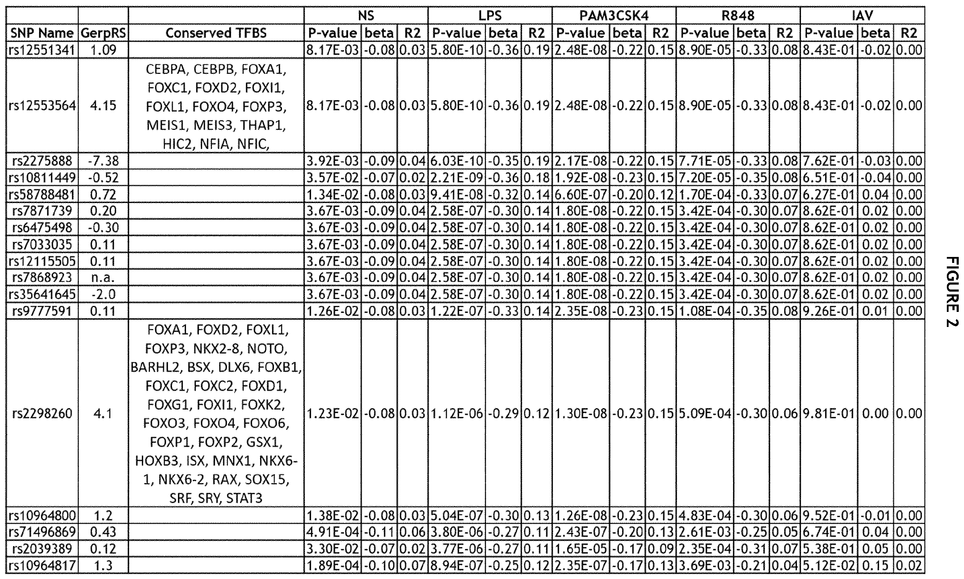

**Figure 2****. Analysis of genetic variants associated with variation of IFNB1 expression in LPS-activated monocytes.**
   Regulatory elements are defined based on the Regulatory Build from Ensembl v80 (32) and transcription factor binding sites (TFBS) are defined based on chip-Seq data from Encode (clustered TFBS peaks v3). GerpRS measures base-wise conservation across mammals (33). A GerpRS>2 indicates conservation, whereas a GerpRS<2 indicates neutral evolution.

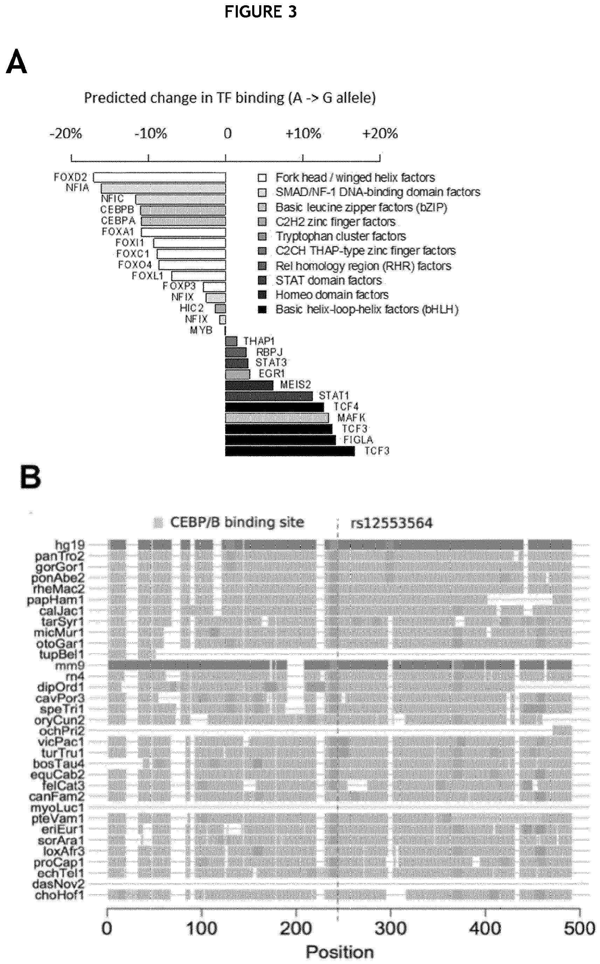

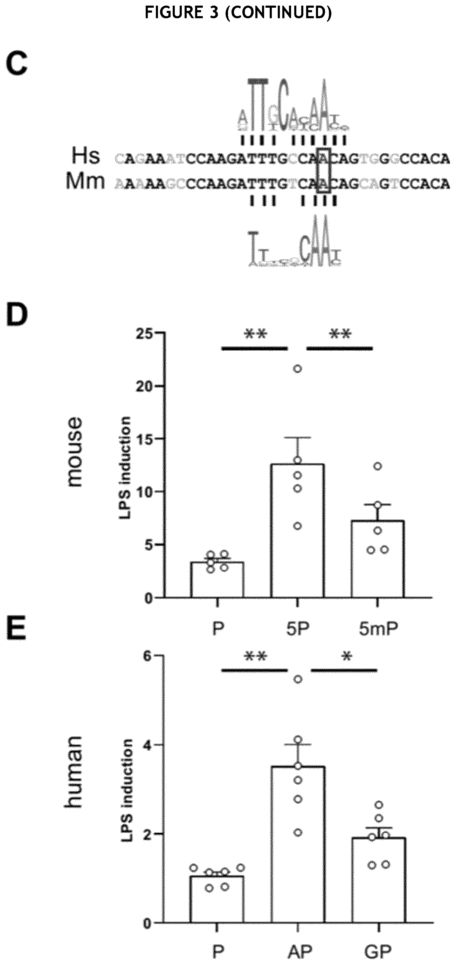

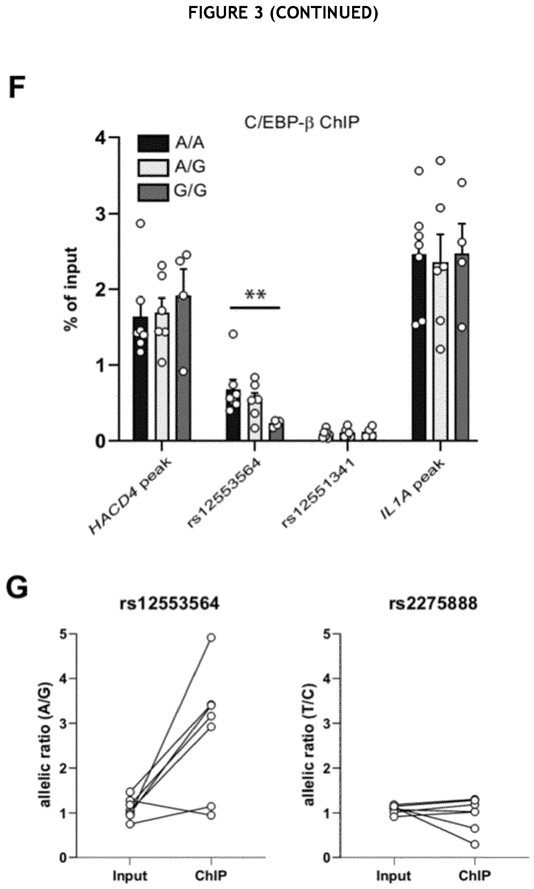

**Figure 3****: Role of C/EBP-B binding in Far IFN Regulatory Enhancer (FIRE) function.**
   (*A*) Predicted impact of rs12553564 on transcription factor binding. Difference in transcription factor binding scores between the derived (G) and ancestral (A) alleles at the rs12553564 locus. Only transcription factors with a binding score > 85% for either the ancestral or derived allele are reported. Transcription factors are colored according to the tertiary structure of their DNA binding domain. (*B*) Cross-species conservation of C/EBP-B binding site at the rs12553564 locus. Sequence alignment of 46 vertebrate species are displayed in a -500 bp window around the rs12553564 variant. For each genome, only sequences aligning to the human or mice genome are shown, and are displayed by grey boxes. In each genome, matches to the human C/EBP-B motif (>85% of maximal score) are highlighted in orange. (*C*) Alignment of the human (Hs) and murine (Mm) sequence around rs12553564 (red box). The Jaspar motifs for human and murine C/EBP-B are indicated above and under the alignment, respectively. (*D*) Plasmids encoding firefly luciferase under the control of the *Ifnb1* promoter (P) flanked or not with FIRE (5P) or the A→G mutated FIRE (5mP) were transfected into RAW264.7 cells together with a plasmid coding for NanoLuc luciferase under the thymidine kinase promoter. After 22 hrs, cells were treated or not with 100 ng/ml LPS. After 30 hrs, luciferase levels were measured. Shown are the ratios of relative luciferase activity between LPS treated versus non-treated cells. Results are presented as mean ± s.e.m with open circles showing individual values, each in triplicate (n=5). *: p<0.05 (two-sided ratio paired t test). (*E*) C/EBP-B binding as revealed by ChIP on the indicated loci in activated macrophages from healthy donors with the indicated genotypes (A/A: 7 donors, A/G: 6 donors, G/G: 4 donors). *HACD4 peak* and *IL1A peak* indicate non-polymorphic C/EBP-B peaks in *HACD4 (PTPLAD2)* and *IL1A,* respectively. Results are presented as mean ± s.e.m with open circles showing individual values. **: p<0.01 (two-sided Mann-Whitney test). (F) Allelic ratio of rs12553564 (left) and rs2275888 (right) before (input) or after (ChIP) C/EBP-B ChIP. ChIP was performed in samples from 7 healthy donors heterozygous for both SNPs. Allelic ratio was determined by allele-specific quantitative Taqman PCR. Connected open circles represent the results for an individual donor.

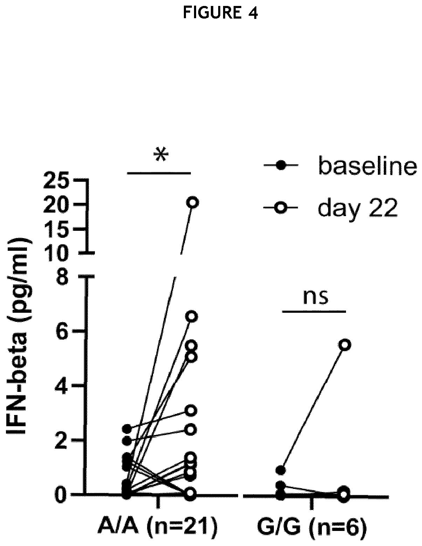

**Figure 4****. Level of IFN-B in patient serum at baseline and 22 days after treatment start.**
   Lung cancer patients were treated with radiotherapy and anti-CTLA-4 (21). IFN-B was assayed in serum samples before treatment (baseline) or 22 days after treatment start (day 22). The rs12553564 SNP was tested in each patient, and IFN-B assay results are presented for patients with homozygous ancestral genotype (left, A/A) or homozygous variant genotype (right, G/G). Statistical significance was determined using a One-tailed paired Wilcoxon test.

### Examples

The present invention is further defined in the following examples. It should be understood that these examples, while indicating preferred embodiments of the invention, are given by way of illustration only. The person skilled in the art will readily understand that these examples are not limitative and that various modifications, substitutions, omissions, and changes may be made without departing from the scope of the invention.

### 1. MATERIAL AND METHODS

### 1.1 eQTL mapping.

Mapping of eQTLs was performed using previously published gene expression and genotype data (34). Briefly, gene expression data from CD14⁺ monocytes was obtained from 200 individuals of either African (N=100) or European (N=100) ancestry, after 6h of stimulation by LPS (N=184 samples) or rest. After correction for batch effects and technical covariates (GC content and 5'/3' bias), log₂-transformed FPKM were used for eQTL mapping. Genotypes were obtained for 9,166 common variants (Minor Allele frequency > 5% in either population) from the *IFNB1* locus (<1Mb from *IFNB1*), based on genotyping with Illumina HumanOmni5-Quad beadchips, exome sequencing using Nextera Rapid Capture Expanded Exome Kits, and imputation with IMPUTE v.2 (35). Only variants that passed stringent quality criteria were kept for analysis. Details on SNP filtering and gene expression pre-processing can be found in (34). To map cis-eQTLs of *IFNB1,* we ran the MatrixEQTL R package (36) on both basal and stimulated gene expression, applying an inverse normal rank transformation for each condition and adjusting for the population of origin. Family wise error rate was estimated based on 1000 permutations, retrieving for each permutation the lowest p-value across all SNPs and conditions and choosing a threshold such that a significant p-value is detected in less than 1% of permutations. To assess the trans-effect of the rs12553564 variant, we focused on the LPS stimulated condition and tested the SNP for association will all 12,578 expressed genes, using a linear model with population as a covariate. P-values were corrected using Benjamini Hochberg correction, and a 1% FDR threshold was applied. We further required a minimal effect size (|β|) of 0.2 to consider associations as significant. GO enrichment analyses were performed with GOseq, using the set of all expressed genes as background (37).

### 1.2 Variant prioritization for follow up.

Peak eQTL was defined as the most significant SNP across 5 conditions, when combining both European and African indivudals. r² of nearby genetic variants with rs12553564 was computed across all individuals (100 of African-descent and 100 of Europeans descent). To annotate genetic variants, we retrieved regulatory elements predictions from the Ensembl Regulatory Build v80 (32), and overlapped them with regulatory variants using the Genomic Ranges R package. Similarly, we retrieved a list of Transcription factor binding sites (TFBS) identified by chip-Seq in the Encode Consortium (clustered TFBS peaks v3), and overlapped candidate snps with TFBS position. Conservation across mammals was assessed using base-wise GerpRS (33), and sites with GerpRS>2 were deemed conserved, whereas a GerpRS<2 indicates neutral evolution. GerpRS base-wise mammalian conservation scores were downloaded from the Sidow lab as a measure of local sequence conservation (http://mendel.stanford.edu/sidowlab/downloads/gerp/hg19.GERP_scores.tar.gz).

### 1.3 Cell culture.

All incubations were performed at 37°C in 5% CO₂ in a humidified atmosphere. Bone Marrow Derived Macrophages (BMDM) were obtained as described (38). Briefly, bone marrow cells flushed from the hind limbs of mice were filtered through a 70 µm cell strainer and incubated for 3 h on cell culture treated Petri dishes (1x10 cm dish per animal) in BMDM medium (Iscove's Modified Dulbecco's Medium (IMDM, ThermoFisher) supplemented with 10% fetal calf serum (FCS, Sigma), 1% Penicillin/Streptomycin (PS, ThermoFisher), and 10 µM thioglycerol (Sigma)). Non-adherent cells were seeded at 3.5×10⁶ cells per dish (10 cm cell culture treated) in BMDM medium supplemented with 25 ng/ml mouse CSF1 (Miltenyi), and incubated for 7 days with complete medium changes at days 3 and 6. Incubation with LPS (Sigma #L4516) were performed for 24 h at 100 ng/ml in BMDM medium supplemented with 2.5% FCS. RAW 264.7, NIH3T3, and EL4 cells were grown in Dulbecco's Modified Eagle's Medium (DMEM, ThermoFisher) supplemented with 10% FCS and 1% PS. In these cell lines, mycoplasma contamination was checked every 6 months with the MycoAlert detection kit (Lonza). Peripheral blood mononuclear cells were prepared by Ficoll density centrifugation (Lymphocytes separation medium, Eurobio) of buffy coats from anonymous healthy donors obtained at the "Etablissement Français du Sang" (EFS), and frozen in SVF supplemented with 10% dimethyl sulfoxide. Approximately 10⁶ cells were retained for genomic DNA purification (PureLink genomic DNA minikit, ThermoFisher), and genotyped for rs12553564 and rs12551341 with snp genotyping taqman assays (ThermoFisher). After thawing and washing, CD14⁺ cells were purified by labelling with CD14 microbeads and magnetic isolation on LS columns (Miltenyi), according to manufacturer's instructions. Purity was checked by CD14-PE (Miltenyi) labelling and analysis on a Guava easyCyte 8HT cytometer (Millipore). They were then directly processed for ChIP, or differentiated into macrophages in the presence of 50 ng/ml human M-CSF (Miltenyi) as described (39).

### 1.4 ChIP.

ChIP experiments were performed as described (40). Briefly, cells were fixed with 1% formaldehyde for 10 min at room temperature, and chromatin was sonicated to 100-500 bp fragments in 1 mM EDTA, 0.5 mM EGTA, 10 mM Tris pH8 with a Bioruptor Pico sonication device (Diagenode). Lysates were precleared with Dynabeads (ThermoFisher), and 1% was sampled as the input. They were then incubated overnight at 4°C with antibodies against mouse CTCF (Millipore #07-729), mouse RAD21 (Abcam ab992), or human C/EBP-β (Abcam ab32358) and then 3 h with saturated Dynabeads. After extensive washing, beads were eluted in 1% sodium dodecyl sulfate, 100 mM NaHCO₃, and decrosslinked overnight at 65°C. The immunoprecipitated DNA was purified and used directly in PCR with primers shown in Table 1 below, or genotyped with allele-specific quantitative Taqman PCR assays (ThermoFisher, rs12553564 assay ID C__252065_10, rs2275888 assay ID C_16087171_10), or processed for next generation sequencing. In the latter case, they were quantified using Qbit fluorometer (Thermofisher). Sequencing libraries were prepared from 1 ng DNA using the MicroPlex kit (Diagenode) according to the manufacturer's protocol. DNA was repaired and end-blunted by enzymatic treatment. Stem-loop adaptors with blocked 5' ends were ligated to the 5' end of the genomic DNA, leaving a nick at the 3' end. The 3' ends of the genomic DNA were extended to complete library synthesis and Illumina-compatible indexes were added through amplification. Libraries were purified using AMPure XP beads (Beckman Coulter) and quantified using Qbit fluorometer. Libraries fragment size distribution was verified using the Bioanalyzer high sensitivity DNA chip (Agilent Technologies). Libraries were mixed in an equimolar pool and a 1% spike-in PhiX Control v3 (Illumina) was added. Clusters were generated and sequenced using a Nextseq 500 instrument (Illumina) in single read mode (75 cycles). Sequences were demultiplexed, quality controlled by the Aozan tool (41), trimmed with Cutadapt 1.5, and aligned on the mm9 version of the mouse genome with Bowtie 2. Peak calling was performed with MACS with default settings, and co-localization of peaks was analyzed with seqMINER (42).

### 1.5 Luciferase assays.

The vector encoding firefly luciferase under the control of the murine *Ifnb1* promoter has been previously described (43), and is based on pGL3-basic. Six DNA fragments of around 500 bp centered on each individual enhancer were obtained by PCR amplification of genomic DNA from WT BMDM with primers designed with Primer3Plus (http://www.bioinformatics.nl/cgi-bin/primer3plus/primer3plus.cgi). They were cloned in front the *Ifnb1* promoter, and sequence-verified. The human *IFNB1* promoter was inserted in front of the luciferase gene in pGL4.12 by Sequence and Ligation Independent Cloning (SLIC). A fragment of human genomic DNA centered on rs12553564 was amplified by PCR from THP-1 genomic DNA (allele A) and inserted in front of the promoter by SLIC, and then mutated to the G allele by SLIC. All constructs were sequenced (Eurofins), and primers can be found in Table 1 below. RAW264.7, NIH-3T3, and EL4 cells were transfected in triplicate with jetPEI-Macrophage (Polyplus Transfection), Lipofectamine 2000 (ThermoFisher), or Lipofectamine 3000 (ThermoFisher), respectively, according to manufacturers' instructions. A vector coding for NanoLuc luciferase under the control of the thymidine kinase promoter (or Renilla luciferase under the control of the CMV promoter for NIH3T3 cells) was used to normalize transfection efficiencies. Thirty hours after transfection, both luciferase activities were measured with the Nano-Glo Dual (or Dual-Glo) Luciferase Assay System (Promega), according to manufacturer's instructions. Where indicated, cells were treated with 100 ng/ml LPS for the last 8 h of incubation. The mean of the values obtained for the promoter alone in n independent experiments was used to normalize individual values. Results are reported as mean ± s.e.m. of n independent experiments.

**Table 1: Primers used.**

| **Use** | **Region amplified** | **SEQ ID NO** | **Sequences of forward and reverse primers** |
|---|---|---|---|
| Mouse enhancer cloning | E1 | 9 | 5'-TGACCAGGTACCATCTGAACTAGTCTTGAAATGCTGGAGGAAT- AATC-3' |
| | | 10 | 5'-TGACATGAATTCGAGCTCGCTAGCTCTTGCACAGTGACTCTATG-3' |
| | E2 | 11 | 5'-TGACCAGGTACCATCTGAACTAGTCTAGATGTTTCGTTTTGCTTAG-3' |
| | | 12 | 5'-TGACATGAATTCGAGCTCGCTAGCGGCTGAGGATTACCTTATTTC-3' |
| | E3 | 13 | 5' - TGACCAGGT ACCATCTGAACT AGTCTCTTCGTTGTTCTGGAA T AAC-3' |
| | | 14 | 5'-TGACATGAATTCGAGCTCGCTAGCTAGAGACCCCAGAAATTCAA- TATG-3' |
| | E4 | 15 | 5'-TGACCAGGTACCATCTGAACTAGTAGCCAAACAGAAAACAAAATC-3' |
| | | 16 | 5'-TGACATGAATTCGAGCTCGCTAGCCACGAAACCAAGTATTACAC-3' |
| | E5 | 17 | 5'-TGACCAGGTACCATCTGAACTAGTTGCTTAAAAGGGCATTTC-3' |
| | | 18 | 5'-TGACATGAATTCGAGCTCGCTAGCAGCTACTTAAATTGCAGTTTAG-3' |
| | E6 | 19 | 5'-TGACCAGGTACCATCTGAACTAGTCTGTTGTGGCTTCGCAATGC-3' |
| | | 20 | 5'-TGACATGAATTCGAGCTCGCTAGCCCTGCGCTATGTAAATCC-3' |
| | E5 mutagenesis (SLIC) | 21 | 5'-GCAGCAGTCCACATGATC-3' |
| | | 22 | 5'-GTGGACTGCTGCTGACAAATCTTGGGCTTT-3' |
| Human enhancer cloning (SLIC) | *IFNB1* promoter | 23 | 5'-CCTGAGCTCGCTAGCCATAGGAAGGACCAACTGTATC-3' |
| | | 24 | 5'-GGCCAGATCTTGATATCCTCCTTTCTCCATGGGTATG-3' |
| | pGL4.12 | 25 | 5'-GGATATCAAGATCTGGCCTC-3' |
| | | 26 | 5'-GGCTAGCGAGCTCAG-3' |
| | hsE5 | 27 | 5'-CCTGAGCTCGCTAGCCAATATATCCTGTACATTCTGTA-3' |
| | | 28 | 5'-ACAGTTGGTCCTTCCTATAATTATAATTGCAGTTCAGTAG-3' |
| | vector | 29 | 5'-ATAGGAAGGACCAACTGTATC-3' |
| | | 30 | 5'-GGCTAGCGAGCTCAG-3' |
| | rs12553564 mutagenesis | 31 | 5'-GCAGTGGGCCACATGATC-3' |
| | | 32 | 5'-TGTGGCCCACTGCTGGCAAATCTTGGATTTC-3' |
| PCR analysis of C/EBP-β ChIP | HACD4 peak | 7 | 5'-GGGGAAACAGGAAAACCAAG-3' |
| | | 8 | 5'-ATTATGGGATGGCGCTGAG-3' |
| | rs12553564 | 1 | 5'-GTCCTGGAAAATCACCTAGTGC-3' |
| | | 2 | 5'-GCCAAACATAGACCCTCTTGAC-3' |
| | rs12551341 | 3 | 5'-GAAAGAGGGAGAGGGGAGTG-3' |
| | | 4 | 5'-GAGTTTTAGTATATCCAAAGAGATGTGC-3' |
| | IL1A peak | 5 | 5'-ATGAGGTGTTGCGTGTCTTG-3' |
| | | 6 | 5'-CGTGACTTCCAAAGTTGCTG-3' |

### 2. RESULTS

### 2.1. A genetic variant is associated with a decreased interferon response in myeloid cells.

We and others have identified single nucleotide polymorphisms (SNPs) in the human population that are associated with differential expression of *IFNB1* in LPS-activated monocytes (34, 44). These SNPs are called expression quantitative trait loci (eQTLs). Because such eQTLs might give indications on DNA regions that regulate expression of *IFNB1,* we precisely mapped them in a region covering 2 Mb around *IFNB1.* We performed this analysis jointly on individuals of African or European descent, in order to break population-specific haplotypic blocks, and allow a finer resolution of the mapping of causal variants (45). The peak of the association was located over the *PTPLAD2* gene, including a set of 17 SNPs significantly correlated with the expression level of *IFNB1* in LPS activated monocytes (**Table 2,** **Fig. 1A**, p < 4.2 × 10⁻⁶, corresponding to a family wise error rate of 1%).

**Table 2: SNPs correlated with the expression level of IFNB1 in LPS activated monocytes.**

| **SNP Name** | **Position on chromosome 9** | **Major allele** | **Minor allele (derived)** | **daf EUB** | **daf AFB** | **r² with eQTL peak** |
|---|---|---|---|---|---|---|
| rs12551341 | 21014629 | T | C | 0.23 | 0.28 | 1.00 |
| rs12553564 | 21017241 | A | G | 0.23 | 0.28 | 1.00 |
| rs2275888 | 21017885 | T | C | 0.23 | 0.30 | 0.96 |
| rs10811449 | 21009192 | G | A | 0.23 | 0.24 | 0.91 |
| rs58788481 | 21018812 | (GC) | (G) | (0.23) | (0.23) | 0.86 |
| rs7871739 | 21020115 | T | A | 0.24 | 0.33 | 0.86 |
| rs6475498 | 21021247 | T | C | 0.24 | 0.33 | 0.86 |
| rs7033035 | 21021386 | A | G | 0.24 | 0.33 | 0.86 |
| rs12115505 | 21022501 | A | C | 0.24 | 0.33 | 0.86 |
| rs7868923 | 21023447 | C | T | 0.24 | 0.33 | 0.86 |
| rs35641645 | 21024671 | G | A | 0.24 | 0.33 | 0.86 |
| rs9777591 | 21021953 | G | A | 0.23 | 0.25 | 0.84 |
| rs2298260 | 21029331 | T | C | 0.24 | 0.29 | 0.75 |
| rs10964800 | 21028637 | C | T | 0.24 | 0.29 | 0.75 |
| rs71496869 | 21020234 | (GAC) | (G) | (0.24) | (0.40) | 0.69 |
| rs2039389 | 20801596 | A | G | 0.29 | 0.33 | 0.35 |
| rs10964817 | 21056086 | C | T | 0.57 | 0.49 | 0.27 |

For each SNP the derived allele is computed based on 6EPO alignments. When the ancestral allele is unknown, the minor allele is provided instead in parentheses. daf: derived allele frequency. When the derived allele is unknown, the minor allele frequency is provided instead. Peak eQTL is defined as the most significant SNP across 5 conditions, r² is computed across all individuals (100 of African descent and 100 of European descent).

The strongest association with *IFNB1* expression was observed for variants rs12553564 and rs12551341(p<5.8 x 10⁻¹⁰, R² = 19%, **Fig. 1*****B*),** which are in perfect linkage disequilibrium (LD). No further association was found between genetic variants and *IFNB1* expression when conditioning on rs12553564 (data not shown), suggesting that variants in low LD with rs12553564 do not contribute to the variability in *IFNB1* expression. Variant rs12553564 is part of a 15 kb haplotypic block containing 15 SNPs in high LD (r² > 0.5), including the previously reported rs2275888 (r² = 0.96) (44). Among these SNPs, rs12553564 was the only one fulfilling a series of analytical criteria (**Fig. 2**)**.** First, rs12553564 is located in a predicted regulatory element as defined by the *Ensembl* v80 database. Second, rs12553564 is overlapped by experimentally defined transcription factor binding sites established by the Encode consortium. Third, the nucleotide affected in rs12553564 is located at a position conserved across mammals as determined with the GERP++ tool (GerpRS score > 2). And fourth, rs12553564 overlaps several transcription factor binding sites that are conserved across mammals.

Variant rs12553564 is an A to G substitution located on chromosome 9 at position 21,017,241 (genome version GRCh38), in the third intron of *PTPLAD2.* We analyzed Hi-C data from the THP-1 human monocytic cell line (46), and found that the *PTPLAD2* region loops to *IFNB1* (**arrow in** **Fig. 1*****C***). This loop is included in a topological domain lined by 2 head-to-tail binding sites for CTCF (**Fig. 1*****C***), a classical organization of functionally isolated chromatin domains (47). The rs12553564 variant was also associated in *trans* with a total of 433 genes (FDR < 0.01, |B_{eQTL}| > 0.2, **Fig. 1*****D***), among which 94% are down-regulated. Gene ontology analysis revealed that antiviral response genes were strongly enriched among them (Fold Enrichment > 10.2, *p* < 1.2 × 10⁻⁴⁸, **Fig. 1*****E***). These genes most probably represent Interferon Stimulated Genes. In addition to LPS, the rs12553564 variant was found to be an eQTL for *IFNB1* in monocytes activated by Pam₃CSK₄ (targeting the TLR1/TLR2 receptors) and R848 (targeting the TLR7/TLR8 receptors). Upon Pam₃CSK₄ activation, but not R848 activation, rs12553564 was also a trans-eQTL for Interferon Stimulated Genes (data not shown). These results show that there is a far human enhancer regulating the expression of IFNB, herein called FIRE (for "Far IFN Regulatory Enhancer") which is polymorphic, and suggest that this enhancer can regulate *IFNB1* expression in myeloid cells in defined conditions of activation.

### 2.2. Role of C/EBP-B binding in FIRE function

The association between *IFNB1* expression and a human polymorphism in FIRE suggested that a single nucleotide substitution was sufficient to affect FIRE enhancer function. We sought to determine whether this could be due to decreased binding of a transcription factor. We first assessed the impact of the rs12553564 variant on transcription factor binding motifs. The A to G substitution was predicted to change the binding of 26 transcription factors on the rs12553564 region (**Fig. 3*****A***). Among factors with predicted decreased binding, only 4 were expressed in monocytes (fragments per kb per millions reads (FPKM) > 1), with the highest transcript levels being observed for CEBPB (not shown), the gene coding for the monocyte/macrophage transcription factor C/EBP-B (48). We analyzed the conservation of C/EBP-B binding motifs in mammals, and found that the rs12553564 C/EBP-B motif was conserved in more than 20 species **(****Fig. 3*****B*).** The G allele modified an adenine in a high probability position of this motif, as shown by the JASPAR (49) sequence logos for human and murine C/EBP-B (**Fig. 3*****C***).

To study the role of this substitution in the enhancer activity of FIRE, we mimicked rs12553564 in the mouse orthologous sequence by mutating the TTTGTCAAC motif to TTTGTCAGC in the luciferase reporter plasmid. This mutation did not modify the constitutive enhancer activity of FIRE (data not shown), but significantly decreased its capacity to be induced by LPS (**Fig. 3*****D***). We also assayed the effect of a 500 bp fragment of human genome centered on rs12553564 and carrying either the A allele or the G allele on the activity of the human *IFNB1* promoter driving expression of firefly luciferase (in pGL4.12). Again, the G allele did not modify the constitutive activity of the enhancer (data not shown), but significantly decreased its capacity to be induced by LPS (**Fig. 3*****E***).

We then assessed the binding of C/EBP-B at the rs12553564 position by performing ChIP experiments in myeloid cells from genotyped healthy donors. In accordance with published results (50), the signal obtained in monocytes was too low to reveal a genotype-dependent binding of C/EBP-B on the rs12553564 locus. However, in activated monocyte-derived macrophages, the binding of C/EBP-B was 3 times higher on the A/A allele of rs12553564 than on the G/G allele (0.67 ± 0.13 % of input *vs* 0.23 ± 0.02, mean ± s.e.m., 7 donors with the A/A genotype vs 4 with G/G genotype, p < 0.01; **Fig. 3*****F***). The binding on the G/G allele was actually not different from a negative control, which was also the case for rs12551341, regardless of the genotype (**Fig. 3*****F***). Finally, the rs12553564 genotype did not influence C/EBP-B binding on nearby or distant non-mutated C/EBP-B binding loci (**Fig. 3*****F***), demonstrating the specificity of the differential binding on the rs12553564 locus. In order to confirm this differential binding, we performed C/EBP-B ChIP in samples from heterozygous donors, genotyped the resulting DNA with an allele-specific rs12553564 quantitative Taqman PCR, and calculated the A/G allelic ratio in the input and after immunoprecipitation (**Fig. 3*****G***). We found an allelic ratio of 1.1 ± 0.9 in the input, which increased to 2.85 ± 0.52 after ChIP (n = 7, mean ± s.e.m., p < 0.05). All these donors were also heterozygous for rs2275888 (T/C allelic ratio of 1.08 ± 0.03 in the input), but immunoprecipitating C/EBP-B did not enrich one allele of rs2275888 against the other (T/C allelic ratio of 0.97 ± 0.14 after ChIP, p = 0.36) (**Fig. 3*****G***). Altogether, these results show that the rs12553564 polymorphism of FIRE prevents binding of the C/EBP-B transcription factor, and inhibits its LPS inducible enhancer activity.

### 2.3. The rs12553564 genotype predicts the increase in IFN-B level in the serum of patients treated by radiotherapy + anti-CTLA4.

In metastatic lung cancer patients treated with radiotherapy and anti CTLA-4, an observable abscopal response is associated with an increase of IFN-B levels in the serum of patients after treatment (21). We determined the genotype of rs12553564 in these patients and analyzed the increase of IFN-B during treatment in homozygous ancestral patients (genotype A/A) and homozygous variant patients (genotype G/G). Genotyping was performed by Taqman PCR on blood-derived DNA samples. We found that 47.6% of the patients with the A/A genotype underwent an increase in blood IFN-B level during treatment (>0.5 pg/ml between day 22 and baseline; 10 out of 21 patients), but only 16.7% of the patients with the G/G genotype (1 out of 6) **(Table 3 and** **Fig. 4****)**

**Table 3. Percentage of IFN responders classified according to their genotype and their IFN-B serum levels**

| | rs12553564 genotype | |
|---|---|---|
| | **A/A** | **G/G** |
| **IFN responders (ΔIFN>0.5 pg/ml)** | **47.6 %** | **16.7 %** |

### Conclusion

Through the molecular analysis of a murine genetic model of IFN-B deregulation in myeloid cells, we have identified a myeloid super-enhancer whose looping to the *IFNB1* gene correlates with increased IFNB1 transcription. This super-enhancer contains one LPS inducible enhancer, whose human ortholog carries an IFNB1 eQTL, i.e. a genetic polymorphism associated with differential IFNB1 expression. The minor allele disrupts a conserved C/EBP-B binding motif, prevents C/EBP-B binding, and results in decreased IFNB1 expression levels in activated monocytes. Mimicking the mutation in the murine enhancer directly inhibits its LPS inducible activity. Our results identify a new myeloid-specific *IFNB1* enhancer whose polymorphism at the rs12553564 SNP controls *IFNB1* expression through binding of C/EBP-B. We have named this enhancer FIRE, for Far *IFNB1* Regulating Enhancer.

FIRE is a new regulatory region of *IFNB1* expression, with the unique property of being tissue-type specific. The fact that the activity of FIRE depends on the binding of C/EBP-B provides a molecular explanation for tissue specificity.

The analysis of different monocyte activation pathways revealed a specific pattern of association with rs12553564. The polymorphism at the rs12553564 locus is furthermore clearly associated with patient responsiveness to treatments dependent on the successful stimulation of an IFN-B dependent adaptive immune response, such as radiotherapy. Indeed, the homozygous G allele results in significantly reduced patient responsiveness to radiotherapy.

### Bibliographic references

1. U. Müller, et al., Functional role of type I and type II interferons in antiviral defense. Science 264, 1918-21 (1994).
2. F. McNab, K. Mayer-Barber, A. Sher, A. Wack, Type I interferons in infectious disease. Nat. Rev. Immunol. 15, 87-103 (2015).
3. J. W. Schoggins, Interferon-stimulated genes and their antiviral effector functions. Curr. Opin. Virol. 1, 519-525 (2011).
4. S. D. Der, A. Zhou, B. R. Williams, R. H. Silverman, Identification of genes differentially regulated by interferon alpha, beta, or gamma using oligonucleotide arrays. Proc. Natl. Acad. Sci. U. S. A. 95, 15623-8 (1998).
5. A. Le Bon, et al., Cross-priming of CD8+ T cells stimulated by virus-induced type I interferon. Nat. Immunol. 4, 1009-1015 (2003).
6. M. Montoya, et al., Type I interferons produced by dendritic cells promote their phenotypic and functional activation. Blood 99, 3263-3271 (2002).
7. R. M. Welsh, K. Bahl, H. D. Marshall, S. L. Urban, Type 1 Interferons and Antiviral CD8 T-Cell Responses. Plos Pathog. 8, e1002352 (2012).
8. T. Agalioti, et al., Ordered recruitment of chromatin modifying and general transcription factors to the IFN-beta promoter. Cell 103, 667-78 (2000).
9. D. Panne, T. Maniatis, S. C. Harrison, An Atomic Model of the Interferon-B Enhanceosome. Cell 129, 1111-1123 (2007).
10. E. Ford, D. Thanos, The transcriptional code of human IFN-beta gene expression. Biochim Biophys Acta 1799, 328-336 (2010).
11. F. G. Goh, et al., Beyond the enhanceosome: cluster of novel κB sites downstream of the human IFN-B gene is essential for lipopolysaccharide-induced gene activation. Blood 116, 5580-8 (2010).
12. L. Zeng, et al., XBP-1 couples endoplasmic reticulum stress to augmented IFN-beta induction via a cis-acting enhancer in macrophages. J Immunol 185, 2324-2330 (2010).
13. M. Klar, J. Bode, Enhanceosome formation over the beta interferon promoter underlies a remote-control mechanism mediated by YY1 and YY2. Mol. Cell. Biol. 25, 10159-70 (2005).
14. T. Josse, et al., Association of the interferon-B gene with pericentromeric heterochromatin is dynamically regulated during virus infection through a YY1-dependent mechanism. Nucleic Acids Res. 40, 4396-4411 (2012).
15. V. Marcato, et al., β-Catenin Upregulates the Constitutive and Virus-Induced Transcriptional Capacity of the Interferon Beta Promoter through T-Cell Factor Binding Sites. Mol. Cell. Biol. 36, 13-29 (2015).
16. A. R. Banerjee, Y. J. Kim, T. H. Kim, A novel virus-inducible enhancer of the interferon-B gene with tightly linked promoter and enhancer activities. Nucleic Acids Res. 42, 12537-12554 (2014).
17. B. C. Burnette, et al., The efficacy of radiotherapy relies upon induction of type I interferon-dependent innate and adaptive immunity. Cancer Res. 71, 2488-2496 (2011).
18. L. Deng, et al., STING-Dependent Cytosolic DNA Sensing Promotes Radiation-Induced Type I Interferon-Dependent Antitumor Immunity in Immunogenic Tumors. Immunity 41, 843-852 (2014).
19. C. Vanpouille-Box, et al., DNA exonuclease Trex1 regulates radiotherapy-induced tumour immunogenicity. Nat. Commun. 8, 15618 (2017).
20. X. Wang, et al., Suppression of Type I IFN Signaling in Tumors Mediates Resistance to Anti-PD-1 Treatment That Can Be Overcome by Radiotherapy. Cancer Res. 77, 839-850 (2017).
21. S. C. Formenti, et al., Radiotherapy induces responses of lung cancer to CTLA-4 blockade. Nat. Med. 24, 1845-1851 (2018).
22. J. Crouse, U. Kalinke, A. Oxenius, Regulation of antiviral T cell responses by type I interferons. Nat. Rev. Immunol. 15, 231-242 (2015).
23. S. Wan, et al. Chemotherapeutics and Radiation Stimulate MHC Class I Expression through Elevated Interferon-beta Signaling in Breast Cancer Cells. PLOS ONE 7(3): e32542 (2012).
24. D. Bentley, S. Balasubramanian, H. Swerdlow, et al. Accurate whole human genome sequencing using reversible terminator chemistry. Nature 456, 53-59 (2008).
25. M. Margulies, M. Egholm, W.E. Altman, et al. Genome sequencing in microfabricated high-density picolitre reactors. Nature. 437(7057), 376-380 (2005).
26. K.J. McKernan, H.E. Peckham, G.L. Costa, et al. Sequence and structural variation in a human genome uncovered by short-read, massively parallel ligation sequencing using two-base encoding. Genome Res. 19(9), 1527-1541 (2009).
27. T.D. Harris et al., Single-Molecule DNA Sequencing of a Viral Genome. Science 320(5872), 106-109 (2008).
28. J. Eid, et al. Real-time DNA sequencing from single polymerase molecules. Science. 323(5910), 133-138 (2009).
29. J. Rothberg, et al. An integrated semiconductor device enabling non-optical genome sequencing. Nature 475, 348-352 (2011).
30. J. Clarke, et al. Continuous base identification for single-molecule nanopore DNA sequencing. Nature Nanotech 4, 265-270 (2009).
31. P.J. Asiello and A.J. Baeumner, Miniaturized isothermal nucleic acid amplification, a review. Lab Chip 11(8), 1420-1430 (2011).
32. D. R. Zerbino, S. P. Wilder, N. Johnson, T. Juettemann, P. R. Flicek, The Ensembl regulatory build. Genome Biol. 16, 56 (2015).
33. E. V. Davydov, et al., Identifying a high fraction of the human genome to be under selective constraint using GERP++. PLoS Comput. Biol. 6, e1001025 (2010).
34. H. Quach, et al., Genetic Adaptation and Neandertal Admixture Shaped the Immune System of Human Populations. Cell 167, 643-656 (2016).
35. B. N. Howie, P. Donnelly, J. Marchini, A Flexible and Accurate Genotype Imputation Method for the Next Generation of Genome-Wide Association Studies. PLoS Genet. 5, e1000529 (2009).
36. A. A. Shabalin, Matrix eQTL: ultra fast eQTL analysis via large matrix operations. Bioinformatics 28, 1353-1358 (2012).
37. M. D. Young, M. J. Wakefield, G. K. Smyth, A. Oshlack, Gene ontology analysis for RNA-seq: accounting for selection bias. Genome Biol. 11, R14 (2010).
38. A. Assouvie, L. P. Daley-Bauer, G. Rousselet, Growing Murine Bone Marrow-Derived Macrophages. Methods Mol. Biol. 1784, 29-33 (2018).
39. A. Kelly, A. M. Grabiec, M. A. Travis, Culture of Human Monocyte-Derived Macrophages. Methods Mol. Biol. 1784, 1-11 (2018).
40. G. Rousselet, Chromatin Immunoprecipitation in Macrophages. Methods Mol. Biol. 1784, 177-186 (2018).
41. S. Perrin, C. Firmo, S. Lemoine, S. Le Crom, L. Jourdren, Aozan: an automated post-sequencing data-processing pipeline. Bioinformatics 33, 2212-2213 (2017).
42. T. Ye, et al., seqMINER: an integrated ChIP-seq data interpretation platform. Nucleic Acids Res. 39, e35 (2011).
43. F. Ferri, et al., TRIM33 switches off Ifnb1 gene transcription during the late phase of macrophage activation. Nat. Commun. 6, 8900 (2015).
44. B. P. Fairfax, et al., Innate Immune Activity Conditions the Effect of Regulatory Variants upon Monocyte Gene Expression. Science (80-. ). 343, 1246949 (2014).
45. X. Wen, F. Luca, R. Pique-Regi, Cross-population joint analysis of eQTLs: fine mapping and functional annotation. Plos Genet. 11, e1005176 (2015).
46. D. H. Phanstiel, et al., Static and Dynamic DNA Loops form AP-1-Bound Activation Hubs during Macrophage Development. Mol. Cell 67, 1037-1048.e6 (2017).
47. Y. Guo, et al., CRISPR Inversion of CTCF Sites Alters Genome Topology and Enhancer/Promoter Function. Cell 162, 900-910 (2015).
48. R. Huber, D. Pietsch, T. Panterodt, K. Brand, Regulation of C/EBPβ and resulting functions in cells of the monocytic lineage. Cell. Signal. 24, 1287-1296 (2012).
49. A. Khan, et al., JASPAR 2018: update of the open-access database of transcription factor binding profiles and its web framework. Nucleic Acids Res. 46, D260-D266 (2018).
50. T.-H. Pham, et al., Dynamic epigenetic enhancer signatures reveal key transcription factors associated with monocytic differentiation states. Blood 119, e161-e171 (2012).

## Claims

1. An *in vitro* method for predicting the efficiency of a treatment stimulating an IFN-B dependent adaptive immune response, comprising a step of detecting the rs12553564 single nucleotide polymorphism (SNP), or an SNP in high linkage disequilibrium with same, said SNP being selected from rs12551341, rs2275888, and rs10811449, in a biological sample of a subject in need thereof.

2. The method of claim 1, wherein the treatment is a radiotherapy, an anti-cancer chemotherapy drug, or an anti-microbial drug, such as an anti-viral drug.

3. The method of claim 1 or 2, wherein the presence of homozygous G alleles of the rs12553564 SNP, homozygous C alleles of the rs12551341 SNP or of the rs2275888 SNP, or homozygous A alleles of the rs10811449 SNP, indicates that the treatment will be less efficient for said subject.

4. The method of any one of claims 1 to 3, wherein the biological sample is a biological fluid, preferably blood, plasma, lymph or saliva.

5. The method of any one of claims 1 to 4, further comprising the steps of:
a) isolating nucleic acid from the biological sample, and
b) optionally, amplifying the nucleic acid,
prior to detecting the SNP.

6. The method of claim 5, wherein amplifying the SNP is performed by PCR with primers of 12 to 30 contiguous nucleotides or by isothermal amplification of said SNP such that the nucleic acid of sequence SEQ ID NO: 34, 36, 38, or 40 is amplified.

7. The method of any one of claims 1 to 6, wherein the SNP is detected by allelic discrimination, preferably by probe hybridization, amplification, sequencing, mass spectrometry, Southern blotting, or two or more thereof.

8. The method of any one of claims 1 to 7, wherein the subject has a cancer.

9. The method of claim 8, wherein said cancer is selected from among breast cancer, colorectal cancer, bladder cancer, liver cancer, pancreatic cancer, lung cancer, cervical cancer, thyroid cancer, leukemia, skin cancer, prostate cancer, stomach cancer, and cancer of the head or neck.

10. Use of a kit containing the means to detect the rs12553564 single nucleotide polymorphism (SNP) or an SNP in high linkage disequilibrium with same, said SNP being selected from rs12551341, rs2275888, and rs10811449, in a nucleic acid, for predicting the efficiency of a treatment stimulating an IFN-B dependent adaptive immune response in a subject in need thereof, preferably wherein said subject is diagnosed with cancer.

11. The use of claim 10, wherein said kit contains reagents for detecting the single nucleotide polymorphism within SEQ ID NO: 33, 35, 37 or 39, wherein said reagents are preferably primers and/or a probe.

12. The use of claim 11, wherein said reagents comprise the primers of SEQ ID NOs: 1 and 2, amplifying a nucleic acid fragment comprising rs12553564.

13. An *in vitro* method of screening a subject diagnosed with cancer for responsiveness to radiotherapy comprising:
a) genotyping the rs12553564 single nucleotide polymorphism, or an SNP in high linkage disequilibrium with same, said SNP being selected from rs12551341, rs2275888, and rs10811449, in a biological sample of the subject, and
b) determining the responsiveness of the subject to radiotherapy,
wherein the presence of a homozygous G allele of the rs12553564 SNP, a homozygous C allele of the rs12551341 SNP or of the rs2275888 SNP, or a homozygous A allele of the rs10811449 SNP, is indicative of non-responsiveness to radiotherapy.

14. The method of claim 13, further comprising:
c) selecting a treatment regimen comprising exogenous IFN-B, exogenous IFN-α, and/or a checkpoint inhibitor drug when a homozygous G allele of the rs12553564 SNP, a homozygous C allele of the rs12551341 SNP or of the rs2275888 SNP, or a homozygous A allele of the rs10811449 SNP is detected.

15. Use of rs12553564, or of an SNP in high linkage disequilibrium with same, said SNP being selected from rs12551341, rs2275888, and rs10811449, as a prognostic marker of responsiveness to a treatment stimulating an IFN-B dependent adaptive immune response.


SEQUENCE LISTING

<110>  COMMISSARIAT A L'ENERGIE ATOMIQUE ET AUX ENERGIES
       ALTERNATIVES

<120>  Method for predicting the efficiency of a treatment stimulating
       an IFN-beta dependent adaptive immune response via detection of a
        single nucleotide polymorphism

<130>  B76302EPD40858

<160>  40

<170>  PatentIn version 3.5

<210>  1
<211>  22
<212>  DNA
<213>  Artificial

<220>
<223>  rs12553564 - forward primer

<400>  1
gtcctggaaa atcacctagt gc                                              22


<210>  2
<211>  22
<212>  DNA
<213>  Artificial

<220>
<223>  rs12553564 - reverse primer

<400>  2
gccaaacata gaccctcttg ac                                              22


<210>  3
<211>  20
<212>  DNA
<213>  Artificial

<220>
<223>  rs12551341 - forward primer

<400>  3
gaaagaggga gaggggagtg                                                 20


<210>  4
<211>  28
<212>  DNA
<213>  Artificial

<220>
<223>  rs12551341 - reverse primer

<400>  4
gagttttagt atatccaaag agatgtgc                                        28

<210>  5
<211>  20
<212>  DNA
<213>  Artificial

<220>
<223>  IL1A peak - forward primer

<400>  5
atgaggtgtt gcgtgtcttg                                                    20


<210>  6
<211>  20
<212>  DNA
<213>  Artificial

<220>
<223>  IL1A peak - reverse primer

<400>  6
cgtgacttcc aaagttgctg                                                    20


<210>  7
<211>  20
<212>  DNA
<213>  Artificial

<220>
<223>  HACD4 peak - forward primer

<400>  7
ggggaaacag gaaaaccaag                                                    20


<210>  8
<211>  19
<212>  DNA
<213>  Artificial

<220>
<223>  HACD4 peak - reverse primer

<400>  8
attatgggat ggcgctgag                                                     19


<210>  9
<211>  47
<212>  DNA
<213>  Artificial

<220>
<223>  E1 - forward primer

<400>  9
tgaccaggta ccatctgaac tagtcttgaa atgctggagg aataatc                      47


<210>  10
<211>  44
<212>  DNA

<213>  Artificial

<220>
<223>  E1 - reverse primer

<400>  10
tgacatgaat tcgagctcgc tagctcttgc acagtgactc tatg                    44


<210>  11
<211>  46
<212>  DNA
<213>  Artificial

<220>
<223>  E2 - forward primer

<400>  11
tgaccaggta ccatctgaac tagtctagat gtttcgtttt gcttag                  46


<210>  12
<211>  45
<212>  DNA
<213>  Artificial

<220>
<223>  E2 - reverse primer

<400>  12
tgacatgaat tcgagctcgc tagcggctga ggattacctt atttc                   45


<210>  13
<211>  46
<212>  DNA
<213>  Artificial

<220>
<223>  E3 - forward primer

<400>  13
tgaccaggta ccatctgaac tagtctcttc gttgttctgg aataac                  46


<210>  14
<211>  48
<212>  DNA
<213>  Artificial

<220>
<223>  E3 - reverse primer

<400>  14
tgacatgaat tcgagctcgc tagctagaga ccccagaaat tcaatatg                48


<210>  15
<211>  45
<212>  DNA
<213>  Artificial

<220>

<223>  E4 - forward primer

<400>  15
tgaccaggta ccatctgaac tagtagccaa acagaaaaca aaatc                    45


<210>  16
<211>  44
<212>  DNA
<213>  Artificial

<220>
<223>  E4 - reverse primer

<400>  16
tgacatgaat tcgagctcgc tagccacgaa accaagtatt acac                     44


<210>  17
<211>  42
<212>  DNA
<213>  Artificial

<220>
<223>  E5 - forward primer

<400>  17
tgaccaggta ccatctgaac tagttgctta aaagggcatt tc                       42


<210>  18
<211>  46
<212>  DNA
<213>  Artificial

<220>
<223>  E5 - reverse primer

<400>  18
tgacatgaat tcgagctcgc tagcagctac ttaaattgca gtttag                   46


<210>  19
<211>  44
<212>  DNA
<213>  Artificial

<220>
<223>  E6 - forward primer

<400>  19
tgaccaggta ccatctgaac tagtctgttg tggcttcgca atgc                     44


<210>  20
<211>  42
<212>  DNA
<213>  Artificial

<220>
<223>  E6 - reverse primer

<400>  20

tgacatgaat tcgagctcgc tagccctgcg ctatgtaaat cc 42

<210> 21
<211> 18
<212> DNA
<213> Artificial

<220>
<223> E5 mutagenesis (SLIC) - forward primer

<400> 21
gcagcagtcc acatgatc 18

<210> 22
<211> 30
<212> DNA
<213> Artificial

<220>
<223> E5 mutagenesis (SLIC) - reverse primer

<400> 22
gtggactgct gctgacaaat cttgggcttt 30

<210> 23
<211> 37
<212> DNA
<213> Artificial

<220>
<223> IFNB1 promoter - forward primer

<400> 23
cctgagctcg ctagccatag gaaggaccaa ctgtatc 37

<210> 24
<211> 37
<212> DNA
<213> Artificial

<220>
<223> IFNB1 promoter - reverse primer

<400> 24
ggccagatct tgatatcctc ctttctccat gggtatg 37

<210> 25
<211> 20
<212> DNA
<213> Artificial

<220>
<223> pGL4.12 - forward primer

<400> 25
ggatatcaag atctggcctc 20

<210>  26
<211>  15
<212>  DNA
<213>  Artificial

<220>
<223>  pGL4.12 - reverse primer

<400>  26
ggctagcgag ctcag                                                        15


<210>  27
<211>  38
<212>  DNA
<213>  Artificial

<220>
<223>  hsE5 - forward primer

<400>  27
cctgagctcg ctagccaata tatcctgtac attctgta                               38


<210>  28
<211>  40
<212>  DNA
<213>  Artificial

<220>
<223>  hsE5 - reverse primer

<400>  28
acagttggtc cttcctataa ttataattgc agttcagtag                             40


<210>  29
<211>  21
<212>  DNA
<213>  Artificial

<220>
<223>  vector - forward primer

<400>  29
ataggaagga ccaactgtat c                                                 21


<210>  30
<211>  15
<212>  DNA
<213>  Artificial

<220>
<223>  vector - reverse primer

<400>  30
ggctagcgag ctcag                                                        15


<210>  31
<211>  18
<212>  DNA

<213>  Artificial

<220>
<223>  rs12553564 mutagenesis - forward primer

<400>  31
gcagtgggcc acatgatc                                                    18


<210>  32
<211>  31
<212>  DNA
<213>  Artificial

<220>
<223>  rs12553564 mutagenesis - reverse primer

<400>  32
tgtggcccac tgctggcaaa tcttggattt c                                     31


<210>  33
<211>  500
<212>  DNA
<213>  Homo sapiens

<400>  33
tatattaata tatcctgtac attctgtatt ttgtgtgttt ttaataaaat gttttacttg      60

ctcttaatgg tcaaaggaaa atatacttaa gattgaacag tcaaattcac agtaaagagg     120

ggaatgattt atatttttct ttattttaaa agtggatcaa ggagaaaaga ctgggataac     180

ctgtgacttg gcatttatag gctgctagtc ctggaaaatc acctagtgcc cagaaatcca     240

agatttgcca rcagtgggcc acatgatcag aaatccacgt gtataagcta tatcagcatg     300

tttacgacat gactacagtg gtgagtcaag agggtctatg tttggctaag gggaattata     360

tatgattcaa accttagtca aaccatggaa gctatctaca tagctgtttt tcttttttca     420

gtctttcttt ataatataat actctattga tttaatttca ataataaatt ctactgaact     480

gcaattataa ttactgaaca                                                 500


<210>  34
<211>  19
<212>  DNA
<213>  Homo sapiens

<400>  34
gatttgccar cagtgggcc                                                   19


<210>  35
<211>  500
<212>  DNA
<213>  Homo sapiens

<400>  35
ccaaaatgca atgaagtact gcacatgcca caacacagag gaaccttgaa aacattatgc      60

taagtaaaag acgcctgtca caagaggcca catattggat gactccattt atatgaactt 120

tccaggatag gcaatccata gagacagcat atgtgtagtt tccaggggct gagggaaaga 180

gggagagggg agtgactgct actgggtata aggtttcttt tatgggtgat caaaatgttc 240

taggatagya gtgatgattg cacatctctt tggatatact aaaactcaca aattttgtat 300

tttaaaaggg tgaattttat ggtatataaa ttatatctta ataaaactgt tattaaaata 360

agaaaggtta agtgatttgc ccaacatcac ttagcaagta agtagtggag gtgggtttca 420

tctagagaga tcttctacct ctatgtccaa aaatcttcct atgacagcat gctacttctt 480

tcagtgcaaa tgttgttttg 500

<210> 36
<211> 19
<212> DNA
<213> Homo sapiens

<400> 36
ctaggatagy agtgatgat 19

<210> 37
<211> 500
<212> DNA
<213> Homo sapiens

<400> 37
acacatacat gaatggatac atgaataaaa tacagatttc taattacatg gctggctact 60

caaaaaactt agacaccttt caataagatg atctctaggg tctcctccag ataaaacatt 120

ctgatactag cccagcccca cctttttgga aacaaggaat ttgggaacag acctggaaac 180

atatatatat ccgaagtctc ctggtttcca gagttgtaag ctatccatga tgtcaggtca 240

tcccttaayt gaagaggaca gaggggaaaa tctcctaaaa gggagagact ggagctgcat 300

gatgaaactc accctgttga aaatcatcac ttcactgtct gtggcaaggt agcgatgaaa 360

tcccagctgc acagaaagca gcaacaggat ggacccccaa accaaaccac aacacacata 420

cacacgagtg aagactccct ctatattcgg tgtcaatcta attcgttaac aaatcatctg 480

atggttattc aactctaact 500

<210> 38
<211> 19
<212> DNA
<213> Homo sapiens

<400> 38
atcccttaay tgaagagga 19

<210> 39
<211> 500
<212> DNA

<213>  Homo sapiens

<400>  39
gttcacaact gttagagcca agcagaggta tgagtagcat cagtgaagga tctgccactg      60

acaccaaata tgcagtttaa ggttaacaca ggccgcacct atgtctctag cattttatca     120

gtataattag ttgattttgt tatttacata agctttgttt ttgtcttata tctgtcagat     180

gggaaattat agttattaaa attattaaaa ttaaagttat cgaaagttta gtaggtttat     240

ttaactgcrc agttttatac tttgggacat ctaacatata cagaaacaca gaaagataaa     300

attacaatta tctgtattcc accatccaga gcattattga catttttgtc atatatatct     360

ctagactatt ttctgcatat accttatttt gtaattgcaa gaataagttt atgaagttaa     420

taaaagaata agggttgtat ggcaaccata taagaacatg gctttcctag aaaagacaag     480

gattagaatt atggaataac                                                 500


<210>  40
<211>  19
<212>  DNA
<213>  Homo sapiens

<400>  40
tttaactgcr cagttttat                                                   19